# EUROPÄISCHE PATENTSCHRIFT

(11) **EP 1 257 361 B1**
(45) Veröffentlichungstag und Bekanntmachung des Hinweises auf die Patenterteilung: **26.11.2003**
(21) Anmeldenummer: 01907517.5
(22) Anmeldetag: 09.02.2001
(51) Int. Cl.: B01J 31/18, B01J 31/22, B01J 31/24, C07C 13/44, C07C 13/64, C07C 13/68, C07C 2/50, C07B 37/12, C07C 45/50, C07C 253/10, C07C 51/14

(54) **VERBINDUNGEN DES PHOSPHORS, ARSENS UND DES ANTIMONS BASIEREND AUF DIARYLANELLIERTEN BICYCLO[2.2.N]-GRUNDKÖRPERN UND DIESE ENTHALTENDE KATALYSATOREN**
PHOSPHOROUS, ARSENIC AND ANTIMONY COMPOUNDS BASED ON DIARYL-ANELLATED BICYCLO[2.2.N] PARENT SUBSTANCES AND CATALYSTS CONTAINING SAME
COMPOSES DU PHOSPHORE, DE L'ARSENIC ET DE L'ANTIMOINE A BASE DE CORPS DE BASE BICYCLO[2.2.N] DIARYLANNELES ET CATALYSEURS LES COMPRENANT

(30) Priorität: 10.02.2000 DE 10005794; 23.10.2000 DE 10052462
(43) Veröffentlichungstag der Anmeldung: 20.11.2002
(73) Patentinhaber: BASF AKTIENGESELLSCHAFT, 67056 Ludwigshafen (DE)
(72) Erfinder: AHLERS, Wolfgang, 67549 Worms (DE); RÖPER, Michael, 67157 Wachenheim (DE); HOFMANN, Peter, 69118 Heidelberg (DE); WARTH, Daniel, C., M., 69121 Heidelberg (DE); PACIELLO, Rocco, 67098 Bad Dürkheim (DE)
(74) Vertreter: Kinzebach, Werner, Dr.
(86) Internationale Anmeldenummer: EP0101422
(87) Internationale Veröffentlichungsnummer: WO01058589

(56) Entgegenhaltungen:
- WO-A-98/30569
- WO-A-98/39345
- DE-A- 19 652 350
- GB-A- 1 254 063
- US-A- 5 817 848
- DATABASE CHEMABS [Online] CHEMICAL ABSTRACTS SERVICE, COLUMBUS, OHIO, US; FU, TAI Y. ET AL: "The synthesis of anthraphos, a conformationally rigid, C2-symmetric diphosphine and the x-ray crystal structure of [Rh(COD)(anthraphos)]BF4" retrieved from STN Database accession no. 122:265491 XP002170785 & TETRAHEDRON LETT. (1994), 35(41), 7593-6 ,
- HILLEBRAND S ET AL: "4,5-Bis(diphenylphosphino)acridine: A New Type of Tridentate Phosphorus-Nitrogen-Phosphorus Ligands" TETRAHEDRON LETTERS,NL,ELSEVIER SCIENCE PUBLISHERS, AMSTERDAM, Bd. 39, Nr. 8, 19. Februar 1998 (1998-02-19), Seiten 813-816, XP004106806 ISSN: 0040-4039

## Beschreibung

Die vorliegende Erfindung betrifft Verbindungen des Phosphors, Arsens und des Antimons, ein Verfahren zu ihrer Herstellung und einen Katalysator, der wenigstens einen Komplex eines Metalls der VIII. Nebengruppe mit einer solchen Verbindung als Liganden umfasst. Die Erfindung betrifft weiterhin die Verwendung dieser Katalysatoren zur Hydroformylierung, Hydrocyanierung, Carbonylierung, Hydrierung, Olefinoligomerisierung und -polymerisierung sowie zur Metathese.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh-, Ir-, Ru-, Pd- oder Pt-Verbindungen bzw. -komplexe eingesetzt, die zur Aktivitäts- und/oder Selektivitätsbeeinflussung mit N- oder P-haltigen Liganden modifiziert sein können. Bei der Hydroformylierungsreaktion kommt es aufgrund der möglichen CO-Anlagerung an jedes der beiden C-Atome einer Doppelbindung zur Bildung von Gemischen isomerer Aldehyde. Zusätzlich kann es auch zu einer Doppelbindungsisomerisierung kommen. In diesen isomeren Gemischen ist der n-Aldehyd häufig gegenüber dem iso-Aldehyd begünstigt, wobei aufgrund der wesentlich größeren technischen Bedeutung der n-Aldehyde eine Optimierung der Hydroformylierungskatalysatoren zur Erzielung einer größeren n-Selektivität angestrebt wird.

Es ist bekannt, bei der Rhodium-Niederdruck-Hydroformylierung phosphorhaltige Liganden zur Stabilisierung und/oder Aktivierung des Katalysatormetalls einzusetzen. Geeignete phosphorhaltige Liganden sind z. B. Phosphine, Phosphinite, Phosphonite, Phosphite, Phosphoramidite, Phosphole und Phosphabenzole. Die derzeit am weitesten verbreiteten Liganden sind Triarylphosphine, wie z. B. Triphenylphosphin und sulfoniertes Triphenylphosphin, da diese unter den Reaktionsbedingungen eine hinreichende Stabilität besitzen. Nachteilig an diesen Liganden ist jedoch, dass im Allgemeinen nur sehr hohe Ligandenüberschüsse zufriedenstellende Ausbeuten insbesondere an linearen Aldehyden liefen. Demgegenüber erlauben Chelatphosphite bereits bei im Allgemeinen sehr geringen Ligandenüberschüssen hohe Ausbeuten an linearen Aldehyden. Nachteilig an diesen Liganden ist jedoch ihre geringe Stabilität, die sich verbunden mit ihren relativ hohen Beschaffungskosten negativ auf die Wirtschaftlichkeit des Hydroformylierungsprozesses auswirkt.

In Beller et al., Journal of Molecular Catalysis A, 104 (1995), Seiten 17-85, werden rhodiumhaltige, phosphinmodifizierte Katalysatoren zur Hydroformylierung von niedrig siedenden Olefinen beschrieben.

Die DE-A-196 523 50 beschreibt Katalysatoren auf Basis von 4,5-Diphosphinoacridin-Liganden. Diese eignen sich zur Katalyse der Kohlenmonoxid-Konvertierung über das Wassergasgleichgewicht. Weiterhin sollen sie sich zur Katalyse der Hydroformylierung, Carbonylierung, Carboxylierung, Hydrierung, Hydrocyanierung, Hydrosilylierung, Polymerisation, Isomerisierung, Kreuzkupplung und Metathese eignen. Letzteres wird in diesem Dokument nicht durch Ausführungsbeispiele belegt. Nachteilig an diesen Liganden ist ihr aufwendiger mehrstufiger Syntheseweg sowie der für eine Rhodium-Chelatisierung ungünstige Bisswinkel.

Haenel et al. beschreiben in Tetrahedron Letters, Band 34, Nr. 13, Seiten 2107ff (1993), in Tetrahedron Letters, Band 36, Nr. 1, Seiten 75ff (1995) und in Chem. Ber. 124, Seite 1705ff (1991) die Synthese von Bis-(diphenylphosphino)chelaten mit Anthracen-, Dibenzofuran-, Dibenzothiophen- und Xanthen-Grundkörpern. Ein Einsatz dieser Verbindungen in der Katalyse wird nicht beschrieben. Nachteilig an diesen Verbindungen ist ihr mehrstufiger Syntheseweg sowie wiederum der für eine Rhodium-Chelatisierung ungünstige Bisswinkel.

Van Leeuwen et al. beschreiben in Organometallics 1995, 14, Seite 3081ff die Synthese von Chelatphosphinen mit Xanthen-Grundkörpern sowie deren Einsatz als Cokatalysatoren in der Rhodium-Niederdruck-Hydroformylierung von α-Olefinen. Nachteilig an diesen Liganden ist die aufwendige Synthese des Xanthen-Grundkörpers sowie die Notwendigkeit zum Einsatz empfindlicher metallorganischer Verbindungen bei der Synthese. Hydroformylierungsverfahren unter Einsatz von Katalysatoren auf Basis dieser Liganden sind daher wirtschaftlich benachteiligt.

Die katalytische Hydrocyanierung zur Herstellung von Nitrilen aus Olefinen besitzt ebenfalls große technische Bedeutung.

In "Applied Homogeneous Catalysis with Organometallic Compounds", Band 1, VCH Weinheim, Seite 465ff wird allgemein die heterogen und homogen katalysierte Addition von Cyanwasserstoff an Olefine beschrieben. Dabei werden vor allem Katalysatoren auf Basis von Phosphin-, Phosphit- und Phosphonit-Komplexen des Nickels und Palladiums verwendet.

C. A. Tolman et al. beschreiben in Organometallics 1984, 3, Seite 33ff die katalytische Hydrocyanierung von Olefinen in Gegenwart von Nickel(0)-Phosphitkomplexen unter spezieller Berücksichtigung der Effekte von Lewis-Säuren auf die Cyanwasserstoffaddition.

In Advances in Catalysis, Band 33, 1985, Academic Press Inc., Seite 1ff wird übersichtsartig die homogen Nickel-katalysierte Hydrocyanierung von Olefinen beschrieben. Als Katalysatoren werden Nickel(0)-Komplexe mit Phosphin- und Phosphitliganden eingesetzt.

Die US-A-5 817 848 offenbart Hydroformylierungs-, Hydrocyanierungs- und Carbonylierungsverfahren mittels eines Katalysators basierend auf einem chelatisierenden Diphosphin-Liganden, welcher ein linear anelliertes System aus drei Ringen mit ein- bis zweifacher Heterosubstitution der Ringmitglieder an den Positionen 9 und 10 (O, S bzw. O, S, NR, P(O)R, SiR2, CR2 möglich) enthält, sowie einem Metall der VIII. Nebengruppe (bevorzugt Rh, Ni, Pd).

Keine der zuvor genannten Literaturstellen beschreibt Katalysatoren, wie Hydroformylierungskatalysatoren oder Hydrocyanierungskatalysatoren, auf Basis von phosphorhaltigen diarylanellierten Bicyclo[2.2.n]-Grundkörpern.

Der vorliegenden Erfindung liegt die Aufgabe zugrunde, neue Verbindungen des Phosphors, Arsens und des Antimons sowie ein Verfahren zu ihrer Herstellung zur Verfügung zu stellen. Diese Verbindungen sollen sich vorzugsweise als Liganden für Übergangsmetallkomplexe von Metallen der VIII. Nebengruppe eignen, um somit neue Katalysatoren auf Basis dieser Metallkomplexe zur Verfügung zu stellen. Vorzugsweise sollen diese Liganden leicht herstellbar sein und/oder ihre Komplexe unter den Reaktionsbedingungen der zu katalysierenden Reaktionen möglichst stabil sein. Vorzugsweise sollen sich die Katalysatoren zur Hydroformylierung, Hydrocyanierung oder Carbonylierung eignen und eine gute katalytische Aktivität aufweisen.

Überraschenderweise wurden nun Verbindungen des Phosphors, Arsens und des Antimons auf Basis von diarylanellierten Bicyclo[2.2.n]-Grundkörpern gefunden, die sich als Liganden für Übergangsmetallkomplexe von Metallen der VIII. Nebengruppe eignen.

Gegenstand der vorliegenden Erfindung sind somit Verbindungen der allgemeinen Formel I 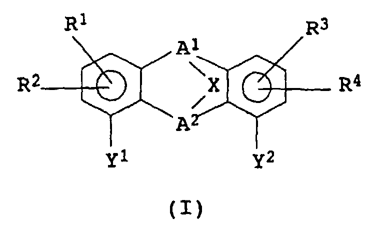 worin
- A¹ und A²: unabhängig voneinander für B, N, P oder CR⁵ stehen, wobei R⁵ für Wasserstoff, Alkyl, Cycloalkyl, Aryl oder Hetaryl oder für einen, gegebenenfalls über eine Spacergruppe gebundenen, polymeren Träger steht,
- X: für O, S oder NR^{a} steht, wobei R^{a} für Wasserstoff, Alkyl, Cycloalkyl oder Aryl steht, oder X für eine C₁- bis C₁₀-Alkylenbrücke steht, die eine, zwei, drei oder vier Doppelbindungen und/oder einen, zwei, drei oder vier Substituenten, die ausgewählt sind unter Alkyl, Alkoxy, Halogen, Nitro, Cyano, Carboxyl, Carboxylat, Cycloalkyl und Aryl, aufweisen kann, wobei der Arylsubstituent zusätzlich einen, zwei oder drei Substituenten, die ausgewählt sind unter Alkyl, Alkoxy, Halogen, Trifluormethyl, -SO₃H, Sulfonat, NE¹E², Alkylen-NE¹E², Nitro, Alkoxycarbonyl oder Cyano, tragen kann, und/oder die Alkylenbrücke X durch ein, zwei oder drei nicht benachbarte, gegebenenfalls substituierte Heteroatome unterbrochen sein kann, und/oder die Alkylenbrücke X ein-, zwei- oder dreifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, -SO₃H, Sulfonat, Nitro, Cyano, Carboxyl, Alkoxycarbonyl, NE¹E² oder Alkylen-NE¹E² tragen können, wobei E¹ und E² gleich oder verschieden sein können und für Alkyl, Cycloalkyl oder Aryl stehen,
- Y¹ und Y²: unabhängig voneinander für mindestens ein Phosphor-, Arsen- oder Antimonatom aufweisende Reste stehen, worin das Phosphor-, Arsen- oder Antimonatom direkt oder über ein Sauerstoffatom an den Phenylring in Formel I gebunden ist,
- R¹, R², R³ und R⁴: unabhängig voneinander für Wasserstoff, Alkyl, Cycloalkyl, Aryl, Alkoxy, Halogen, NE¹E², Alkylen-NE¹E², Trifluormethyl, Nitro, Alkoxycarbonyl oder Cyano stehen, wobei E¹ und E² gleich oder verschieden sind und für Alkyl, Cycloalkyl oder Aryl stehen.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugterweise C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, -SO₃H, Sulfonat, NE¹E², Alkylen-NE¹E², Nitro, Cyano oder Halogen auf. Ein bevorzugter substituierter Arylrest ist auch Pentafluorphenyl.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, -SO₃H, Sulfonat, NE¹E², Alkylen-NE¹E², Trifluormethyl oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² stehen vorzugsweise für N,N-Dimethyl, N,N-Diethyl, N,N-Dipropyl, N,N-Diisopropyl, N,N-Di-n-butyl, N,N-Di-t.-butyl, N,N-Dicyclohexyl oder N,N-Diphenyl.

Halogen steht für Fluor, Chlor, Brom und Iod, bevorzugt für Fluor, Chlor und Brom.

Carboxylat steht im Rahmen dieser Erfindung vorzugsweise für ein Derivat einer Carbonsäurefunktion, insbesondere für ein Metallcarboxylat, eine Carbonsäureesterfunktion oder eine Carbonsäureamidfunktion, besonders bevorzugt für eine Carbonsäureesterfunktion. Dazu zählen z.B. die Ester mit C₁-C₄-Alkanolen, wie Methanol, Ethanol, n-Propanol, Isopropanol, n-Butanol, sec.-Butanol und tert.-Butanol.

A¹ und A² sind vorzugsweise ausgewählt unter N und CR⁵. Wenn R⁵ für Alkyl, Cycloalkyl, Aryl oder Hetaryl steht, so können diese Reste wenigstens einen Substituenten aufweisen, ausgewählt aus den polaren (hydrophilen) Gruppen COOR^{k}, COO-M⁺, SO₃R^{k}, SO₃⁻M⁺, NE³E⁴, NE³E⁴E⁵⁺X⁻, OR^{f}, SR^{f}, (CHR¹CH₂O)ₓR^{k} oder (CH₂CH₂N(E³))ₓR^{k}, worin R^{k}, E³, E⁴ und E⁵ jeweils gleiche oder verschiedene Reste, ausgewählt unter Wasserstoff, Alkyl, Cycloalkyl oder Aryl bedeuten; R¹ für Wasserstoff, Methyl oder Ethyl steht; M⁺ für ein Kation, wie Li⁺, Na⁺, K⁺, NH₄⁺, steht; X⁻ für ein Anion, wie Cl- und Br- steht, und x für eine ganze Zahl von 1 bis 120 steht.

Wenn R⁵ für einen, gegebenenfalls über eine Spacergruppe gebundenen, polymeren Träger steht, so ist der Träger vorzugsweise ausgewählt unter Styrolhomo- und -copolymeren, insbesondere Styrol-Divinylbenzol-Copolymeren (Merrifield-Harzen), Polyamiden, aminomethylierten Polystyrolharzen etc. Geeignete Spacer umfassen Alkylenketten, die gegebenenfalls durch ein oder mehrere nicht benachbarte Heteroatome, wie O, S, NR^{x}, worin R^{x} für Wasserstoff, Alkyl, Cycloalkyl oder Aryl steht, unterbrochen sein können. Weiterhin können die Alkylenketten auch funktionelle Gruppen, wie Ester- und/oder Aminogruppen aufweisen.

Nach einer bevorzugten Ausführung steht R⁵ für Wasserstoff oder C₁-C₈-Alkyl.

Y¹ und Y² stehen vorzugsweise für ein Phosphoratom aufweisende Reste und insbesondere für einen Rest der Formeln PR⁶R⁷, P(OR⁶)R⁷, P(OR⁶)(OR⁷), OPR⁶R⁷, OP(OR⁶)R⁷ oder OP(OR⁶)(OR⁷), worin
- R⁶ und R⁷: unabhängig voneinander für Alkyl, Cycloalkyl, Aryl oder Hetaryl stehen, welche gegebenenfalls einen, zwei oder drei Substituenten ausgewählt unter Alkyl, Cycloalkyl, Aryl, Alkoxy, Cycloalkyloxy, Aryloxy, Halogen, Trifluormethyl, Nitro, Cyano, Carboxyl, Carboxylat, Acyl, -SO₃H, Sulfonat, NE¹E² und Alkylen-NE¹E² tragen, wobei E¹ und E² gleich oder verschieden sind und ausgewählt sind unter Alkyl, Cycloalkyl und Aryl, oder
R⁶ und R⁷ zusammen mit dem Phosphoratom und, falls vorhanden, dem/den Sauerstoffatom(en), an die sie gebunden sind, für einen 5- bis 8-gliedrigen Heterocyclus stehen, der gegebenenfalls zusätzlich ein-, zwei- oder dreifach mit Cycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, Nitro, Cyano, Carboxyl, Carboxylat, -SO₃H, Sulfonat, NE¹E² und Alkylen-NE¹E² tragen können und wobei der Heterocyclus gegebenenfalls zusätzlich ein oder zwei Heteroatom(e), die ausgewählt sind unter N, O und S, aufweisen kann.

Der Rest X steht vorzugsweise für eine C₁- bis C₈-Alkylenbrücke, die, in Abhängigkeit von der Kohlenstoffatomanzahl, 1-, 2- oder 3fach mit Aryl anelliert ist und/oder die 1, 2, 3 oder 4 Substituenten, die ausgewählt sind unter Alkyl, Cycloalkyl und gegebenenfalls substituiertem Aryl, aufweisen kann und/oder die zusätzlich durch 1, 2 oder 3 gegebenenfalls substituierte Heteroatome unterbrochen sein kann.

Bei den anellierten Arylen der Reste X handelt es sich bevorzugt um Benzol oder Naphthalin, insbesondere um Benzol. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE¹E², Alkylen-NE¹E², Trifluormethyl, Nitro, Carboxyl, Alkoxycarbonyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Dieser steht dann bevorzugt für Alkyl oder Alkoxycarbonyl. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy. Alkoxycarbonyl steht vorzugsweise für C₁- bis C₄-Alkoxycarbonyl. Halogen steht dabei insbesondere für Fluor und Chlor.

Wenn die Alkylenbrücke des Restes X durch 1, 2 oder 3, gegebenenfalls substituierte Heteroatome unterbrochen ist, so sind diese bevorzugt ausgewählt unter O, S oder NR¹⁷, wobei R¹⁷ für Alkyl, Cycloalkyl oder Aryl steht.

wenn die Alkylenbrücke des Restes X substituiert ist, so weist sie 1, 2, 3 oder 4 Substituenten auf, der/die vorzugsweise ausgewählt ist/sind unter Alkyl, Cycloalkyl und Aryl, wobei der Arylsubstituent zusätzlich 1, 2 oder 3 Substituenten, die ausgewählt sind unter Alkyl, Alkoxy, Halogen, -SO₃H, Sulfonat, NE¹E², Alkylen-NE¹E², Trifluormethyl, Nitro, Alkoxycarbonyl und Cyano, tragen kann. Vorzugsweise sind die Substituenten der Alkylenbrücke X ausgewählt unter Methyl, Ethyl, n- Propyl, Isopropyl, n-Butyl, sec.-Butyl, tert.-Butyl, Phenyl, p-(C₁- bis C₄-Alkyl)phenyl, bevorzugt p-Methylphenyl, p-(C₁- bis C₄-Alkoxy)phenyl, bevorzugt p-Methoxyphenyl, p-Halogenphenyl, bevorzugt p-Chlorphenyl und p-Trifluormethylphenyl.

Nach einer bevorzugten Ausführungsform steht X für eine nichtanellierte C₁- bis C₃-Alkylenbrücke, wobei C₂- und C₃-Alkylenbrücken gegebenenfalls eine Doppelbindung aufweisen können. Insbesondere weisen diese Reste X 1, 2, 3 oder 4 Substituenten auf, die ausgewählt sind unter Methyl, Ethyl, n-Propyl, iso-Propyl, n-Butyl, sec.-Butyl, tert.-Butyl, Halogen oder Alkyloxycarbonyl.

Nach einer weiteren bevorzugten Ausführungsform ist X ausgewählt unter Resten der Formeln II.1 bis II.10 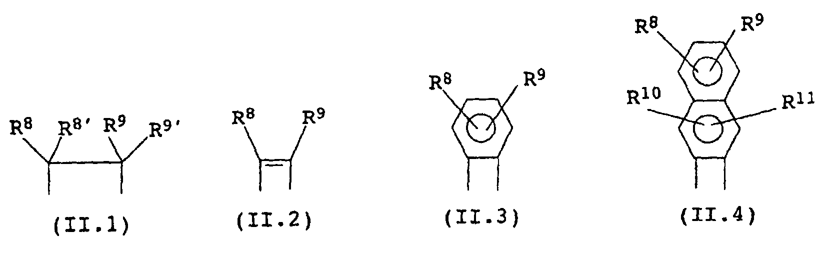 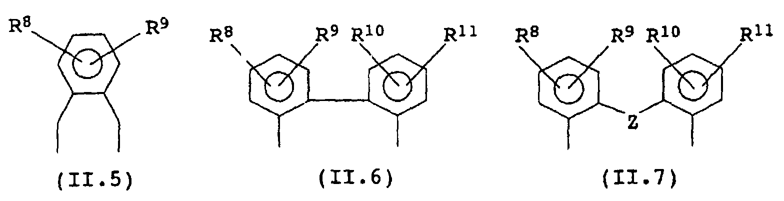 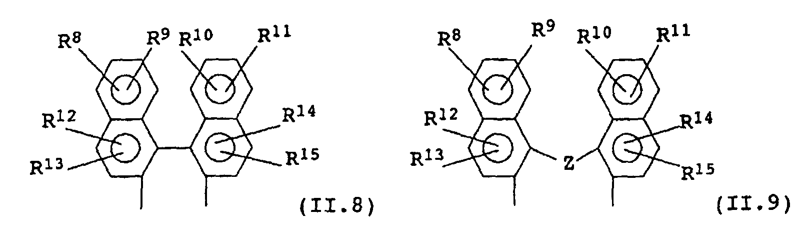 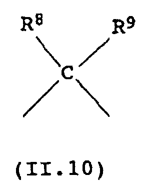 worin
- Z: für O, S oder NR¹⁶ steht, wobei R¹⁶ für Alkyl, Cycloalkyl oder Aryl steht,
oder Z für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei der für Aryl genannten Substituenten, tragen kann,
oder Z für eine C₂- bis C₃-Alkylenbrücke steht, die durch O, S oder NR¹⁶ unterbrochen ist,
- R⁸, R^{8'}, R⁹, R^{9'}, R¹⁰, R¹¹, R¹², R¹³, R¹⁴ und R¹⁵: unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen.

Vorzugsweise steht X für einen Rest der Formeln II.1 oder II.2, worin R⁸ und R⁹ unabhängig voneinander für Wasserstoff oder C₁-C₄-Alkoxycarbonyl, insbesondere COOEt, stehen. Besonders bevorzugt stehen R⁸ und R⁹ beide für Wasserstoff.

Vorzugsweise steht X für einen Rest der Formel II.3, worin R⁸ und R⁹ unabhängig voneinander für Wasserstoff oder C₁-C₄-Alkyl stehen.

Bevorzugt ist in der Formel I einer der Reste Y¹ oder Y² oder sind beide Reste Y¹ und Y² ausgewählt unter solchen Resten der Formeln PR⁶R⁷, P(OR⁶)R⁷, P(OR⁶)(OR⁷), OPR⁶R⁷, OP(OR⁶)R⁷ und OP(OR⁶)(OR⁷), worin R⁶ und R⁷ ausgewählt sind unter C₁-C₆-Alkyl, insbesondere Ethyl, n-Propyl, Isopropyl und tert.-Butyl, C₅-C₇-Cycloalkyl, insbesondere Cyclohexyl, Aryl, insbesondere Phenyl und Hetaryl, insbesondere Pyrrolyl, Pyrazolyl, Imidazolyl, Indolyl und Carbazolyl.

Bevorzugt ist in der Formel I einer der Reste Y¹ oder Y² oder sind beide Reste Y¹ und Y² ausgewählt unter solchen Resten der Formeln PR⁶R⁷, P(OR⁶)R⁷, P(OR⁶)(OR⁷), OPR⁶R⁷, OP(OR⁶)R⁷ und OP(OR⁶)(OR⁷), worin R⁶ und R⁷ zusammen mit dem Phosphoratom und, falls vorhanden, dem/den Sauerstoffatom(en), an die sie gebunden sind, für einen 5- bis 8-gliedrigen Heterocyclus stehen, der gegebenenfalls zusätzlich ein-, zwei- oder dreifach mit Cycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, Nitro, Cyano, Carboxyl, SO₃H, Sulfonat, NE¹E², Alkylen-NE¹E² und Carboxylat, tragen können.

Bevorzugt sind die Reste Y¹ und Y² ausgewählt unter Phosphin-, Phosphinit-, Phosphonit- und/oder Phosphitresten der allgemeinen Formel III 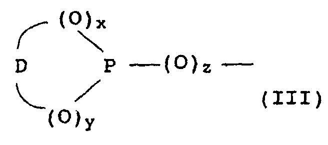 worin
- x, y und z: unabhängig voneinander für 0 oder 1 stehen und
- D: zusammen mit dem Phosphoratom und, falls x und/oder y für 1 stehen, dem/den Sauerstoffatom(en), an die es gebunden ist, für einen 5- bis 8-gliedrigen Heterocyclus steht, der gegebenenfalls zusätzlich ein-, zwei- oder dreifach mit Cycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, SO₃H, Sulfonat, NE¹E², Alkylen-NE¹E², Nitro, Cyano, Carboxyl und Carboxylat, tragen können.

Der Rest D steht vorzugsweise für eine C₂- bis C₇-Alkylenbrücke, die 1-, 2- oder 3-fach mit Aryl anelliert ist und die zusätzlich einen Substituenten, der ausgewählt ist unter Alkyl, Cycloalkyl und gegebenenfalls substituiertem Aryl, aufweisen kann und/oder die zusätzlich durch ein gegebenenfalls substituiertes Heteroatom unterbrochen sein kann.

Bei den anellierten Arylen der Reste D handelt es sich bevorzugt um Benzol oder Naphthalin. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE¹E², Alkylen-NE¹E², Trifluormethyl, Nitro, Carboxyl, Alkoxycarbonyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Anellierte Naphthaline, die im anellierten Ring substituiert sind, weisen vorzugsweise einen Substituenten in ortho-Position zur Phosphonitgruppe auf. Dieser steht dann bevorzugt für Alkyl oder Alkoxycarbonyl. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy. Alkoxycarbonyl steht vorzugsweise für C₁- bis C₄-Alkoxycarbonyl. Halogen steht dabei insbesondere für Fluor und Chlor.

Wenn die C₂- bis C₇-Alkylenbrücke des Restes D durch 1, 2 oder 3, gegebenenfalls substituierte Heteroatome unterbrochen ist, so sind diese ausgewählt unter O, S oder NR¹⁸, wobei R¹⁸ für Alkyl, Cycloalkyl oder Aryl steht. Vorzugsweise ist die C₂- bis C₇-Alkylenbrücke des Restes D durch ein gegebenenfalls substituiertes Heteroatom unterbrochen.

Wenn die C₂- bis C₇-Alkylenbrücke des Restes D substituiert ist, so weist sie 1, 2 oder 3, insbesondere 1 Substituenten auf, der/die ausgewählt ist/sind unter Alkyl, Cycloalkyl und Aryl, wobei der Arylsubstituent 1, 2 oder 3 der für Aryl genannten Substituenten tragen kann. Vorzugsweise weist die Alkylenbrücke D einen Substituenten auf, der ausgewählt ist unter Methyl, Ethyl, Isopropyl, Phenyl, p-(C₁- bis C₄-Alkyl)phenyl, bevorzugt p-Methylphenyl, p-(C₁- bis C₄-Alkoxy)phenyl, bevorzugt p-Methoxyphenyl, p-Halogenphenyl, bevorzugt p-Chlorphenyl und p-Trifluormethylphenyl.

Vorzugsweise steht der Rest D für eine C₄- bis C₇-Alkylenbrücke, die wie zuvor beschrieben anelliert und/oder substituiert und/oder durch gegebenenfalls substituierte Heteroatome unterbrochen ist. Insbesondere steht der Rest D für eine C₄- bis C₅-Alkylenbrücke, die ein- oder zweifach mit Phenyl und/oder Naphthyl anelliert ist, wobei die Phenyl- oder Naphthylgruppen 1, 2 oder 3, insbesondere 1 oder 2 der zuvor genannten Substituenten tragen können.

Vorzugsweise steht der Rest D (d. h. R⁶ und R⁷ gemeinsam) zusammen mit dem Phosphoratom und, falls vorhanden, dem/den Sauerstoffatom(en), an die er gebunden ist, für einen 5- bis 8-gliedrigen Heterocyclus, wobei D (R⁶ und R⁷ gemeinsam) für einen Rest steht, der ausgewählt ist unter den Resten der Formeln II.5 bis II.9, 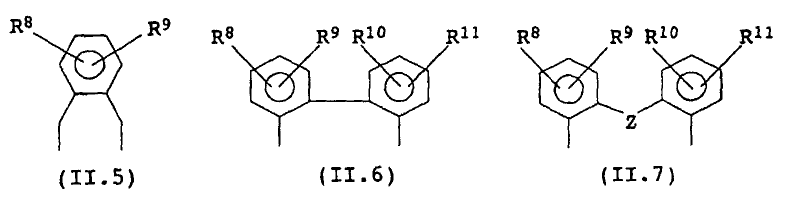 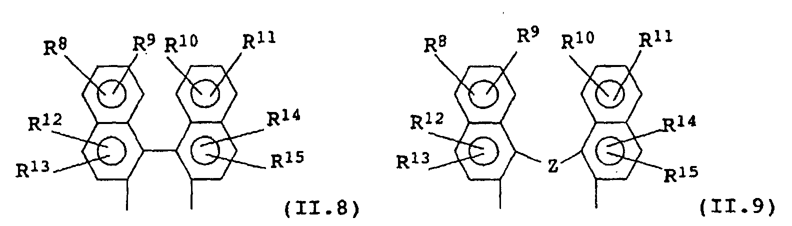 worin
- Z: für O, S oder NR¹⁶ steht, wobei R¹⁶ für Alkyl, Cycloalkyl oder Aryl steht,
oder Z für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei der für Aryl genannten Substituenten tragen kann,
oder Z für eine C₂- bis C₃-Alkylenbrücke steht, die durch O, S oder NR¹⁶ unterbrochen ist,
- R⁸, R⁹, R¹⁰, R¹¹, R¹², R¹³, R¹⁴ und R¹⁵: unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen.

Vorzugsweise steht D für einen Rest der Formel II.5, worin R⁸ und R⁹ für Wasserstoff stehen.

Vorzugsweise steht D für einen Rest der Formel II.6a 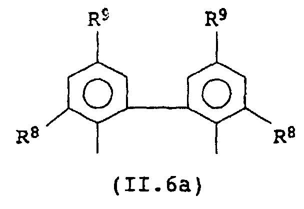 worin
- R⁸: für Wasserstoff, C₁- bis C₄-Alkyl, SO₃H, Sulfonat, NE¹E², Alkylen-NE¹E², vorzugsweise Wasserstoff oder C₁- bis C₄-Alkyl, insbesondere Methyl, Isopropyl oder tert.-Butyl, steht,
- R⁹: für Wasserstoff, C₁- bis C₄-Alkyl, bevorzugt Methyl, Isopropyl oder tert.-Butyl, C₁- bis C₄-Alkoxy, bevorzugt Methoxy, Fluor, Chlor oder Trifluormethyl, steht. R⁹ kann auch für SO₃H, Sulfonat, NE¹E² oder Alkylen-NE¹E² stehen.

Vorzugsweise steht D für einen Rest der Formel II.7a 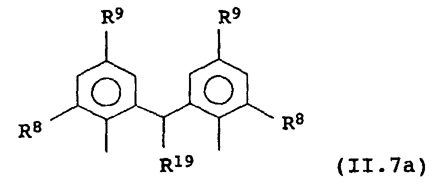 worin
- R⁸ und R⁹: die zuvor bei der Formel II.6a angegebenen Bedeutungen besitzen,
- R¹⁹: für Wasserstoff, C₁- bis C₄-Alkyl, bevorzugt Methyl oder Ethyl, Phenyl, p-(C₁- bis C₄-Alkoxy)phenyl, bevorzugt p-Methoxyphenyl, p-Fluorphenyl, p-Chlorphenyl oder p-(Trifluormethyl)phenyl steht.

Vorzugsweise steht D für einen Rest der Formel II.8, worin R⁸, R⁹, R¹⁰, R¹¹, R¹², R¹³, R¹⁴ und R¹⁵ für Wasserstoff stehen.

Vorzugsweise steht D für einen Rest der Formel II.8, worin R⁸, R⁹, R¹⁰, R¹¹, R¹³ und R¹⁵ für Wasserstoff stehen und die Reste R¹² und R¹⁴ unabhängig voneinander für Alkoxycarbonyl, bevorzugt Methoxy-, Ethoxy-, n-Propyloxy- oder Isopropyloxycarbonyl, stehen. Insbesondere stehen die Reste R¹² und R¹⁴ in ortho-Position zum Phosphoratom bzw. Sauerstoffatom.

Vorzugsweise steht D für einen Rest der Formel II.9, worin R⁸, R⁹, R¹⁰, R¹¹, R¹², R¹³, R¹⁴ und R¹⁵ für Wasserstoff stehen und Z für CR¹⁹ steht, wobei R¹⁹ die zuvor angegebenen Bedeutungen besitzt.

Vorzugsweise steht D für einen Rest der Formel II.9, worin R⁸, R⁹, R¹⁰, R¹¹, R¹³ und R¹⁵ für Wasserstoff stehen, Z für CR¹⁹ steht und die Reste R¹² und R¹⁴ unabhängig voneinander für Alkoxycarbonyl, bevorzugt Methoxy-, Ethoxy-, n-Propyloxy- oder Isopropyloxycarbonyl, stehen. Insbesondere stehen die Reste R¹² und R¹⁴ in ortho-Position zum Phosphoratom bzw. Sauerstoffatom.

Insbesondere ist die phosphorhaltige Verbindung ausgewählt unter Verbindungen der Formeln I.i bis I.v 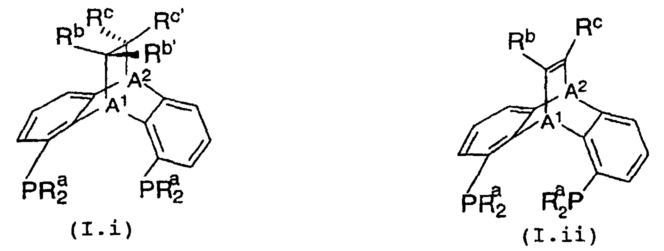 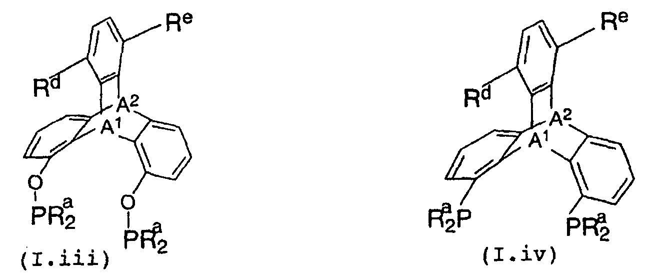 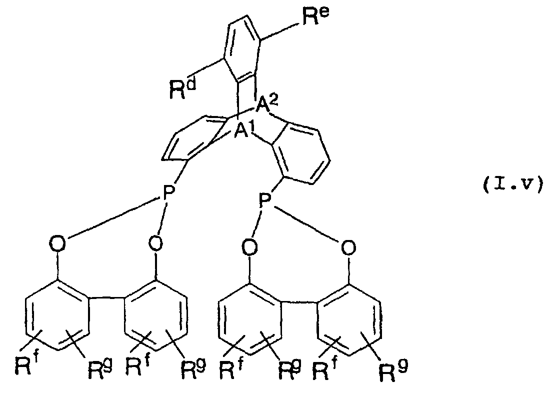 worin
- R^{a}: ausgewählt ist unter C₁-C₆-Alkyl, C₁-C₆-Alkoxy, C₅-C₇-Cycloalkoxy, Phenyl, Phenoxy und Pentafluorphenyl, wobei die Phenyl- und Phenoxyreste gegebenenfalls einen Substituenten, ausgewählt unter Carboxyl, Carboxylat, -SO₃H und Sulfonat aufweisen können,
- R^{b}, R^{b'}, R^{c} und R^{c'}: unabhängig voneinander ausgewählt sind unter Wasserstoff, C₁-C₆-Alkyl, C₁-C₆-Alkoxy, C₁-C₆-Alkoxycarbonyl und Aryl,
- R^{d} und R^{e}: unabhängig voneinander ausgewählt sind unter Wasserstoff und C₁-C₆-Alkyl,
- R^{f} und R^{g}: unabhängig voneinander ausgewählt sind unter Wasserstoff, C₁-C₆-Alkyl und C₁-C₆-Alkoxy, und
- A¹ und A²: unabhängig voneinander für N oder CR⁵ stehen, wobei R⁵ für Wasserstoff oder C₁-C₈-Alkyl steht.

Die Reste R^{b}, R^{b'}, R^{c} und R^{c'} sind vorzugsweise ausgewählt unter Wasserstoff, Methyl, Ethyl, n-Propyl, Isopropyl, n-Butyl, tert.-Butyl, Methoxy, Ethoxy, n-Propoxy, Isopropoxy, n-Butoxy, tert.-Butoxy, Methoxycarbonyl, Ethoxycarbonyl und Phenyl.

Im Folgenden sind Beispiele für erfindungsgemäß bevorzugte phosphorhaltige Verbindungen angegeben.

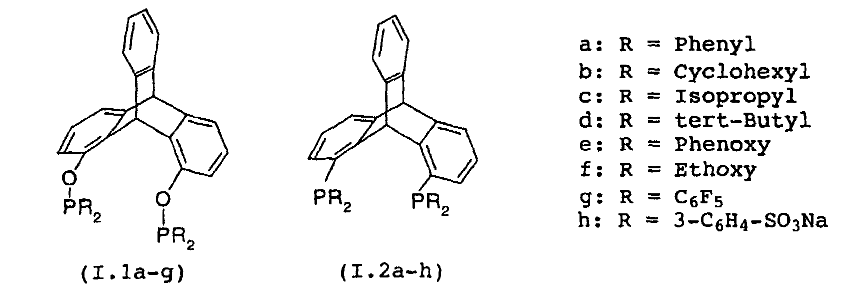

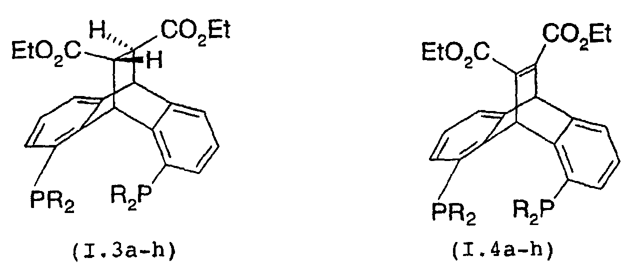

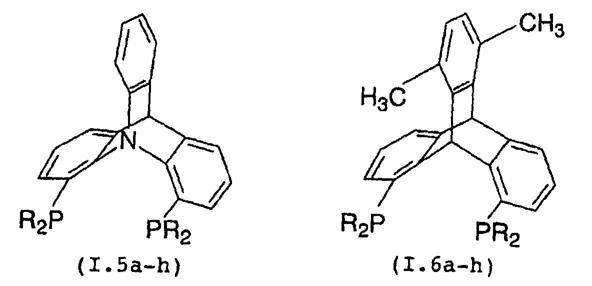

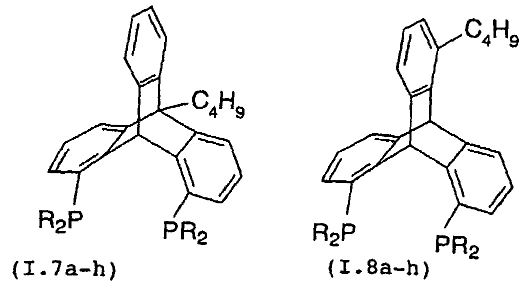

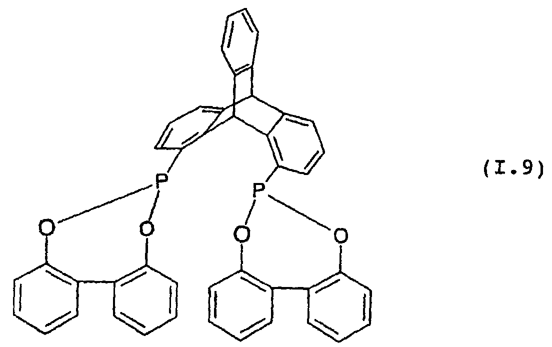

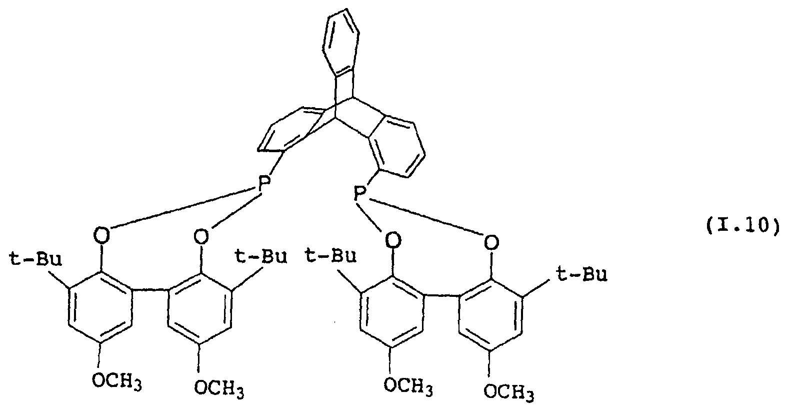

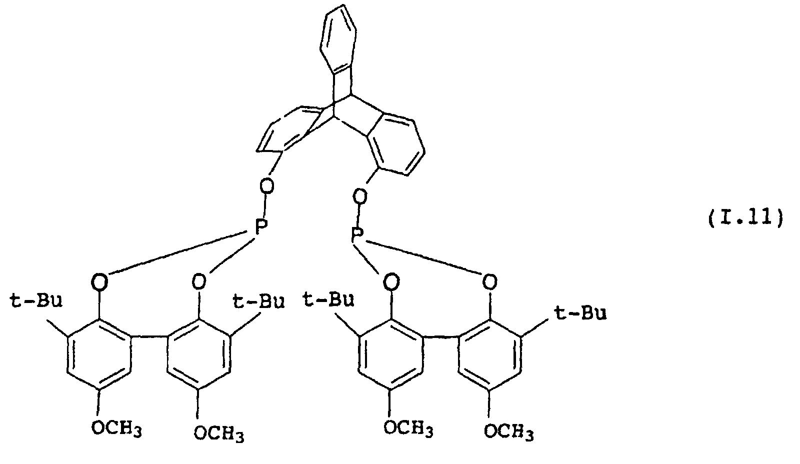

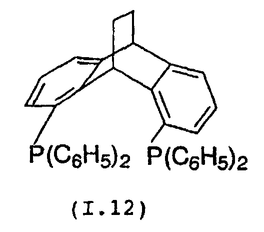

Die Herstellung der erfindungsgemäßen Verbindungen erfolgt in der Regel durch [4+2]-Cycloadditionsreaktionen, im Falle der Verbindungen I mit Bicyclo[2.2.2]-Grundkörpern vorzugsweise durch [4+2]-Cycloaddition (Diels-Alder-Reaktion), von entsprechend substituierten Anthracenen, Acridinen oder von heteroanalogen Verbindungen als Dien-Komponente mit zur Ausbildung der verbrückenden Gruppen X befähigten Dienophilen.

Ein weiterer Gegenstand der Erfindung ist daher ein Verfahren zur Herstellung von Verbindungen der allgemeinen Formel I, wobei X für einen Rest der Formeln 11.1, II.2, II.3 oder II.4 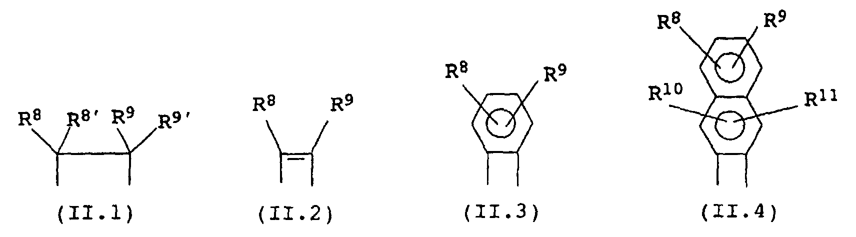 steht, worin
- R⁸, R^{8'}, R⁹, R^{9'}, R¹⁰ und R¹¹: unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen,
durch Umsetzung einer Verbindung der allgemeinen Formel I.1 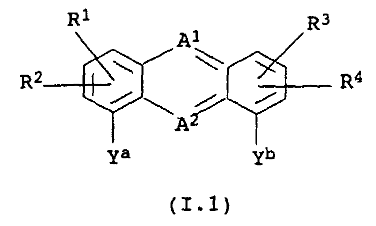 worin
- A¹ und A²: unabhängig voneinander für B, N, P oder CR⁵ stehen, wobei R⁵ für Wasserstoff, Alkyl, Cycloalkyl, Aryl, Hetaryl oder einen, gegebenenfalls über eine Spacergruppe gebundenen, polymeren Träger steht,
- Y^{a} und Y^{b}: unabhängig voneinander für Reste Y¹ oder Y², wie zuvor definiert, stehen,
oder Y^{a} und Y^{b} unabhängig voneinander für Halogen, OH, OC(O)CF₃ oder SO₃Me mit Me = Wasserstoff, Li, Na oder K, stehen, wobei Y^{a} und/oder Y^{b} auch für Wasserstoff stehen kann, wenn jeweils einer der Reste R² und/oder R⁴ eine Alkoxygruppe bedeutet, die sich in der ortho-Position von Y^{a} und/oder Y^{b} befindet,
- R¹, R², R³ und R⁴: unabhängig voneinander für Wasserstoff, Alkyl, Cycloalkyl, Aryl, Alkoxy, Halogen, Trifluormethyl, Nitro, Alkoxycarbonyl oder Cyano stehen,
mit einer Verbindung, ausgewählt unter Verbindungen der Formeln II.a, II.b, II.c oder II.d 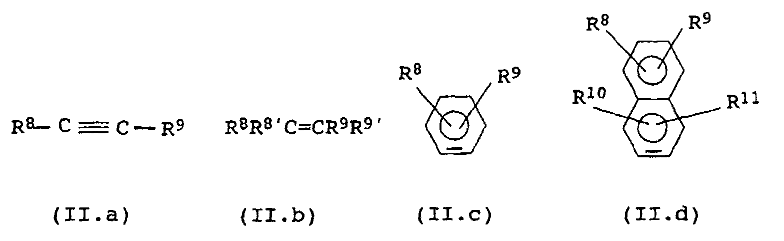 oder einer zur Ausbildung einer Verbindung der Formeln II.c oder II.d geeigneten Vorstufe, in einer [4+2]-Cycloaddition (Diels-Alder-Reaktion) und gegebenenfalls Funktionalisierung von Resten Y^{a} und/oder Y^{b} zu Resten Y¹ und/oder Y².

Zur Herstellung der erfindungsgemäß eingesetzten Verbindungen der Formel I.1, worin R¹, R², R³ und R⁴ die zuvor angegebenen Bedeutungen besitzen, kann man z. B. ein 1,8-Dihalogen-substituiertes Anthrachinon der Formel III, worin Y^{a} und Y^{b} eine der vorgenannten Bedeutungen haben und beispielsweise für Halogen, insbesondere Chlor oder Brom, stehen, gemäß folgendem Schema 1 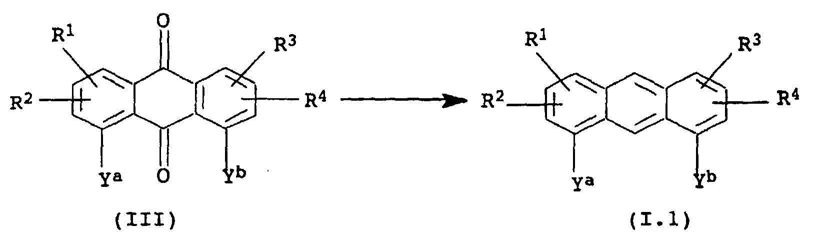 zu einer Verbindung der Formel I.1 reduzieren. Derartige Reduktionen gelingen beispielsweise mit Zink und Ammoniak und anschließender Umsetzung mit HCl/Isopropanol, wie dies z. B. in J. Org. Chem. 1980, 45, 1807-1817; J. Org. Chem. 1973, 38, 1167-1173; Bull. Chem. Soc. Jpn. 1971, 44, 1649-1652 und J. Am. Chem. Soc. 1969, 34, 3089-3092 beschrieben ist. Die Umwandlung von Verbindung III in Verbindung I.1 gelingt beispielsweise auch durch Umsetzung von III mit Natriumborhydrid und Wasser (siehe T.R. Criswell et al., J. Org. Chem. 39 (1974), 770-774) oder durch Umsetzung mit Aluminiumalkoholaten cyclischer Alkohole z.B. Cyclohexanol in Gegenwart dieser Alkohole (siehe M.W. Haenel et al., Chem. Ber. 124, 1991, 333, sowie Fieser, Williamson, Organic Experiments, 3rd ed., 1975, S. 416ff. Ein geeignetes Verfahren zur Herstellung von 1,8-Dichloranthracen findet sich in H. House, J. Org. Chem. 1986, 51, 921-929.

Beispiele für geeignete Verbindungen der Formel III sind z. B. Verbindungen der Formel III mit R¹, R², R³ und R⁴ = Wasserstoff, in denen Ya und Yb ausgewählt sind unter Fluor, Chlor, Brom, Iod SO₃H, SO₃K, SO₃Na, OH, Alkoxy, wie Methoxy oder Ethoxy. Wenn zwei Reste R² und R⁴ oder R¹ und R³ eine Alkoxygruppe bedeuten, die jeweils in ortho-Position zu Y^{a} bzw. Y^{b} stehen, kann Y^{a} und Y^{b} in III auch Wasserstoff bedeuten.

Zum Aufbau des Bicyclo[2.2.2]-Grundgerüstes kann man die Verbindung der Formel I.1, wie zuvor beschrieben, mit einem geeigneten Dienophil, vorzugsweise ausgewählt unter Verbindungen der Formeln II.a, II.b, II.c und II.d, gemäß folgendem Schema 2 umsetzen.

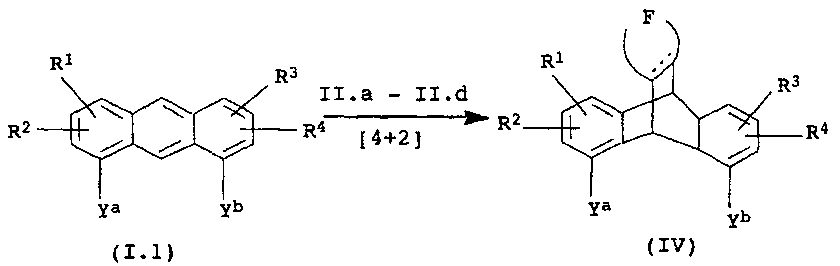

In der Formel IV steht F für einen von einer Verbindung II.a bis II.d nach Entfernen einer Etheno- bzw. Ethinogruppe abgeleiteten Rest. Wird zur Diels-Alder-Reaktion ein Acetylenderivat II.a eingesetzt, so resultieren Verbindungen IV, die eine Ethenobrücke aufweisen (dargestellt durch die gestrichelte Doppelbindung).

Geeignete Verbindungen der Formel II.a sind z. B. Acetylendicarbonsäure und deren Halbester und Diester mit C₁- bis C₈-Monoalkoholen.

Geeignete Verbindungen der Formel II.b sind z. B. Maleinsäure, Fumarsäure sowie deren Halbester und Diester mit C₁- bis C₈-Monoalkoholen.

Die Dehydroaromaten II.c und II.d werden vorzugsweise aus geeigneten Vorstufen in situ hergestellt und zur [4.2]-Cycloaddition eingesetzt. Geeignete Ausgangsstufen und Verfahren sind z. B. in Carey, Sundberg, Advanced Organic Chemistry, 2. Auflage, Teil B, S. 402ff, Plenum Press, New York (1983) beschrieben, worauf hier Bezug genommen wird. Vorzugsweise erfolgt die Herstellung der Dehydroaromaten II.c und II.d durch Diazotierung der entsprechenden ortho-Aminocarbonsäuren gemäß folgendem Schema 3.

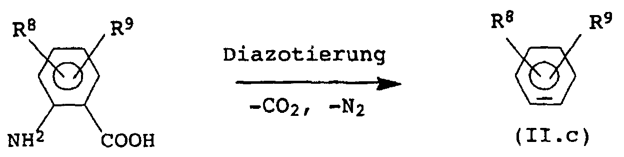

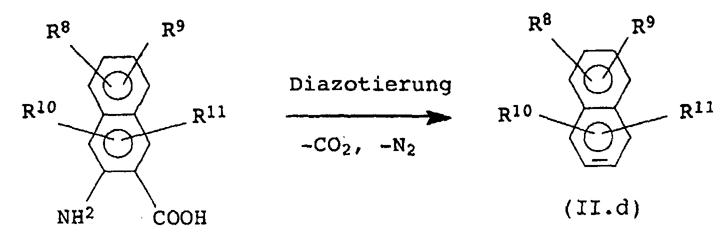

Zur Diazotierung werden vorzugsweise Alkylnitrite, wie n-Butylnitrit, eingesetzt.

Ein geeignetes Verfahren zur Herstellung von 1,8-disubstituierten Ethanoanthracenen, wie z. B. 1,8-Dichlorethanoanthracen, ist in JACS 94 (1972), S. 3080-3088 beschrieben, worauf hier Bezug genommen wird.

Die Funktionalisierung der Reste Y^{a} und Y^{b} zu den Resten Y¹ und Y² kann in Analogie zu bekannten Verfahren erfolgen. Beispielsweise kann man Verbindungen der Formel IV, worin Y^{a} und Y^{b} für Halogen, vorzugsweise Chlor, stehen, zunächst lithiieren und das dabei gebildete Zwischenprodukt mit einer Verbindung, die am Phosphoratom ein Halogenatom, vorzugsweise ein Chloratom trägt, beispielsweise eine Verbindung der Formel Cl-P(R⁶)₂, Cl-P(R⁶)(R⁷) oder Cl-P(R⁷)₂, umsetzen. Die Herstellung der erfindungsgemäß besonders bevorzugten Verbindungen I, worin R für einen Rest der Formel III mit z = 0 steht, erfolgt beispielsweise durch Umsetzung von IV mit Verbindungen der Formel V gemäß folgendem Schema 4, 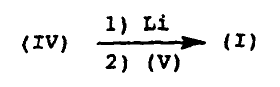 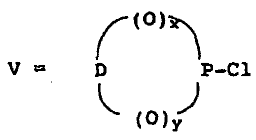 wobei x, y und D die zuvor für die Verbindungen der Formel III angegebenen Bedeutungen besitzen.

Anstelle von Verbindungen der Formel IV mit Y^{a} = Y^{b} = Halogen könen auch solche Verbindungen IV mit Y^{a} = Y^{b} = Wasserstoff lithiiert werden, in denen in der Orthoposition von Y^{a} und Y^{b} sich jeweils eine Alkoxygruppe oder Alkoxycarbonylgruppe befindet. Derartige Reaktionen sind unter dem Begriff "ortho-Lithiierung" in der Literatur beschrieben (siehe z.B. D. W. Slocum, J. Org. Chem., 1976, 41, 3652-3654; J. M. Mallan, R. L. Bebb, Chem. Rev., 1969, 693ff; V. Snieckus, Chem. Rev., 1980, 6, 879-933). Die dabei erhaltenen Organolithiumverbindungen können dann mit den Phosphorhalogenverbindungen in der oben angegebenen Weise zu den Zielverbindungen I umgesetzt werden.

In analoger Weise gelingt die Herstellung der Arsen- und der Antimonverbindungen I.

Verbindungen der Formel IV mit Y^{a} = Y^{b} = OH können durch sukzessive Umsetzung mit Trifluormethansulfonsäureanhydrid in Gegenwart einer Stickstoffbase wie Triethylamin und nachfolgend mit einem Phosphid der Formel MeP(R⁶)₂, worin Me für Li, Na oder K steht, in Verbindungen der Formel I mit Y^{a} = Y^{b} = P(R⁶)₂ umgewandelt werden. Dabei ist R⁶ unter solchen vorgenannten Resten ausgewählt, die gegenüber Basen inert sind. MeP(R⁶)₂ steht dann beispielsweise für KP(C₆H₅)₂. Diese Reaktion kann analog der von J. V. Allen et al. in Tetrahedron 50, 1994, S. 799-808 beschriebenen Umsetzung durchgeführt werden.

Verbindungen IV mit Y^{a} = Y^{b} = SO₃K oder = SO₃Na können durch Umsetzung mit einem Phosphid der Formel MeP(R⁶)₂, worin R⁶ unter solchen vorgenannten Resten ausgewählt ist, die gegenüber Basen inert sind, und Me für Li, Na oder K steht, beispielsweise mit KP(C₆H₅)₂ in die Verbindungen der Formel I mit Y^{a} = Y^{b} = P(R⁶)₂, umgewandelt werden. Diese Reaktion kann analog den von M. W. Haenel, Chem. Ber. 124, 1991, S.333 oder von H. Zorn, Chem. Ber. 98, 1965, S. 2431 beschriebenen Umsetzungen durchgeführt werden.

Verbindungen der Formel IV mit Y^{a} = Y^{b} = F können analog der von Haenel in Synlett, 1988, 301-302 beschriebenen Reaktion in die entsprechenden Diarylphosphine überführt werden.

Ein weiterer Gegenstand der Erfindung ist ein Katalysator, umfassend wenigstens einen Komplex eines Metalls der VIII. Nebengruppe mit mindestens einem P-, As- oder Sb-haltigen Liganden, der ausgewählt ist unter Verbindungen der allgemeinen Formel I, wie zuvor beschrieben.

Vorzugsweise ist das Metall der VIII. Nebengruppe ausgewählt unter Cobalt, Ruthenium, Iridium, Rhodium, Nickel, Palladium und Platin.

Die erfindungsgemäßen Katalysatoren können einen oder mehrere der phosphorhaltigen Verbindungen der Formel I aufweisen. Zusätzlich zu den zuvor beschriebenen Liganden der allgemeinen Formel I können sie noch wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit und Phosphitliganden aufweisen.

Der vorliegenden Erfindung liegt auch die Aufgabe zugrunde, ein verbessertes Verfahren zur Hydroformylierung von Verbindungen, die wenigstens eine ethylenisch ungesättigte Doppelbindung enthalten, zur Verfügung zu stellen. Dabei soll bei der Hydroformylierung von α-Olefinen vorzugsweise ein möglichst hoher Anteil an α-Aldehyden, bzw. -Alkoholen erzielt werden. Insbesondere soll das Verfahren zur Hydroformylierung interner linearer Olefine bei hoher Regioselektivität zugunsten terminaler Produktaldehyde geeignet sein.

Ein weiterer Gegenstand der Erfindung ist ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart wenigstens eines Hydroformylierungskatalysatoren, der ausgewählt ist unter den zuvor beschriebenen erfindungsgemäßen Katalysatoren.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für eine erfindungsgemäße phosphorhaltige Verbindung und q, x, y, z für ganze Zahlen, abhängig von der Wertigkeit und Art des Metalls sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 2 bis 5. Dabei können die Komplexe gewünschtenfalls zusätzlich noch mindestens einen der zuvor beschriebenen weiteren Liganden aufweisen.

Bei dem Metall M handelt es sich vorzugsweise um Cobalt, Ruthenium, Rhodium, Nickel, Palladium, Platin, Osmium oder Iridium und insbesondere um Cobalt, Ruthenium, Iridium, Rhodium, Nickel, Palladium und Platin.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man z. B. wenigstens eine phosphorhaltige Verbindung der allgemeinen Formel I, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltacetat, Cobaltethylhexanoat, Cobaltnaphthanoat, sowie der Cobalt-Caprolactamat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weitere geeignete Verbindungen des Cobalts, Rhodiums, Rutheniums und Iridiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z. B. BF₃, AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Folgeprodukte der Aldehyde. Weitere Lösungsmittel sind Ester aliphatischer Carbonsäuren mit Alkanolen, beispielsweise Essigester oder Texanol™, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch sogenannten "Ionic Liquids" verwendet werden. Hierbei handelt es sich um flüssige Salze, beispielsweise um N,N'-Dialkylimidzoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z.B. die Tetrafluoroborate, Acetate, Tetrachloroaluminate, Hexafluorophosphate, Chloride und Tosylate.

weiterhin ist es möglich die Umsetzungen auch in Wasser oder wässrigen Lösungsmittelsystemen, die neben Wasser ein mit Wasser mischbares Lösungsmittel, beispielsweise einen Alkohol wie Methanol, Ethanol, n-Propanol, Isopropanol, n-Butanol, Isobutanol, ein Keton wie Aceton und Methylethylketon oder ein anderes Lösungsmittel enthalten. Zu diesem Zweck setzt man Liganden der Formel I ein, die mit polaren Gruppen, beispielsweise ionischen Gruppen wie SO₃Me, CO₂Me mit Me = Na, K oder NH₄ oder wie N(CH₃)₄⁺ modifiziert sind. Die Umsetzungen erfolgen dann im Sinne einer zweiphasenkatalyse, wobei der Katalysator sich in der wässrigen Phase befindet und Einsatzstoffe und Produkte die organische Phase bilden. Auch die Umsetzung in den "Ionic Liquids" kann als zweiphasenkatalyse ausgestaltet sein.

Das Molmengenverhältnis von phosphorhaltigem Ligand zu Metall der VIII. Nebengruppe liegt im Allgemeinen in einem Bereich von etwa 1:1 bis 1 000:1.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen etc.

Geeignete geradkettige interne Olefine sind vorzugsweise C₄- bis C₂₀-Olefine, wie 2-Buten, 2-Penten, 2-Hexen, 3-Hexen, 2-Hepten, 3-Hepten, 2-Octen, 3-Octen, 4-Octen etc.

Geeignete verzweigte, interne Olefine sind vorzugsweise C₄- bis C₂₀-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅- bis C₈-Cycloalkene, wie Cyclopenten, Cyclohexen, Cyclohepten, Cycloocten und deren Derivate, wie z. B. deren C₁- bis C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₃- bis C₂₀-Alkenole, -Alkendiole und -Alkadienole, wie 2,7-Octadienol-1. Ein bevorzugtes Alkenol ist Allylalkohol. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclooctatrien sowie Butadienhomo- und -copolymere.

Bevorzugt ist ein Verfahren, das dadurch gekennzeichnet ist, dass der Hydroformylierungskatalysator in situ hergestellt wird, wobei man mindestens eine erfindungsgemäße P-, As- oder Sb-haltige Verbindung, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erf indungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von phosphorhaltigen Verbindungen eine Umsetzung in einem Bereich niedriger Drücke, wie etwa im Bereich von 1 bis 100 bar.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Vorteilhafterweise zeigen die erfindungsgemäßen Katalysatoren eine hohe Aktivität, so dass in der Regel die entsprechenden Aldehyde in guten Ausbeuten erhalten werden. Bei der Hydroformylierung von α-Olefinen sowie von innenständigen, linearen Olefinen zeigen sie zudem eine sehr geringe Selektivität zum Hydrierprodukt des eingesetzten Olefins. Die Hydroformylierungsaktivität von Katalysatoren auf Basis von Verbindungen der Formel I ist überraschenderweise in der Regel höher als die Isomerisierungsaktivität bezüglich der Bildung mittenständiger Doppelbindungen. Vorteilhafterweise zeigen die erfindungsgemäßen Katalysatoren bei der Hydroformylierung von α-Olefinen eine hohe Selektivität zugunsten der α-Aldehyde bzw. -Alkohole. Zudem werden im Allgemeinen auch bei der Hydroformylierung von internen linearen Olefinen (isomerisierende Hydroformylierung) gute Ausbeuten an α-Aldehyden bzw. -Alkoholen und insbesondere auch an n-Aldehyden bzw. -Alkoholen erhalten.

Die zuvor beschriebenen, erfindungsgemäßen Katalysatoren, die chirale Verbindungen der allgemeinen Formel I umfassen, eignen sich zur enantioselektiven Hydroformylierung.

Ein weiterer Gegenstand der Erfindung ist die Verwendung der beschriebenen Katalysatoren, umfassend eine der zuvor beschriebenen P-, As- oder Sb-haltigen Verbindungen, zur Hydroformylierung von Verbindungen mit wenigstens einer ethylenisch ungesättigten Doppelbindung.

Ein weiteres Einsatzgebiet für die erfindungsgemäßen Katalysatoren stellt die Hydrocyanierung von Olefinen dar. Auch die erfindungsgemäßen Hydrocyanierungskatalysatoren umfassen Komplexe eines Metalls der VIII. Nebengruppe, insbesondere Cobalt, Nickel, Ruthenium, Rhodium, Palladium, Platin, bevorzugt Nickel, Palladium und Platin und ganz besonders bevorzugt Nickel. In der Regel liegt das Metall im erfindungsgemäßen Metallkomplex nullwertig vor. Die Herstellung der Metallkomplexe kann, wie bereits für den Einsatz als Hydroformylierungskatalysatoren zuvor beschrieben, erfolgen. Gleiches gilt für die in situ-Herstellung der erfindungsgemäßen Hydrocyanierungskatalysatoren.

Ein zur Herstellung eines Hydrocyanierungskatalysators geeigneter Nickelkomplex ist z. B. Bis(1,5-cyclooctadien)nickel(0).

Gegebenenfalls können die Hydrocyanierungskatalysatoren, analog zu dem bei den Hydroformylierungskatalysatoren beschriebenen Verfahren, in situ hergestellt werden.

Ein weiterer Gegenstand der Erfindung ist daher ein Verfahren zur Herstellung von Nitrilen durch katalytische Hydrocyanierung, das dadurch gekennzeichnet ist, dass die Hydrocyanierung in Gegenwart mindestens eines der zuvor beschriebenen erfindungsgemäßen Katalysatoren erfolgt. Geeignete Olefine für die Hydrocyanierung sind allgemein die zuvor als Einsatzstoffe für die Hydroformylierung genannten Olefine. Eine spezielle Ausführungsform des erfindungsgemäßen Verfahrens betrifft die Herstellung von Gemischen monoolefinischer C₅-Mononitrile mit nichtkonjugierter C=C- und C≡N-Bindung durch katalytische Hydrocyanierung von 1,3-Butadien oder 1,3-Butadien-haltigen Kohlenwasserstoffgemischen und die Isomerisierung/Weiterreaktion zu gesättigten C₄-Dinitrilen, vorzugsweise Adipodinitril in Gegenwart mindestens eines erfindungsgemäßen Katalysators. Bei der Verwendung von Kohlenwasserstoffgemischen zur Herstellung von monoolefinischer C₅-Mononitrilen nach dem erfindungsgemäßen Verfahren wird vorzugsweise ein Kohlenwasserstoffgemisch eingesetzt, das einen 1,3-Butadiengehalt von mindestens 10 Vol.-%, bevorzugt mindestens 25 Vol.-%, insbesondere mindestens 40 Vol.-%, aufweist.

1,3-Butadien-haltige Kohlenwasserstoffgemische sind in großtechnischem Maßstab erhältlich. So fällt z. B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entf ällt. Diese Ströme enthalten immer auch geringe Anteile von im Allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

Die erfindungsgemäßen Katalysatoren lassen sich vorteilhaft zur Hydrocyanierung solcher olefinhaltiger, insbesondere 1,3-Butadien-haltiger Kohlenwasserstoffgemische einsetzen, in der Regel auch ohne vorherige destillative Aufreinigung des Kohlenwasserstoffgemischs. Möglicherweise enthaltene, die Effektivität der Katalysatoren beeinträchtigende Olefine, wie z. B. Alkine oder Cumulene, können gegebenenfalls vor der Hydrocyanierung durch selektive Hydrierung aus dem Kohlenwasserstoffgemisch entfernt werden. Geeignete Verfahren zur selektiven Hydrierung sind dem Fachmann bekannt.

Die erfindungsgemäße Hydrocyanierung kann kontinuierlich, semikontinuierlich oder diskontinuierlich erfolgen. Geeignete Reaktoren für die kontinuierliche Umsetzung sind dem Fachmann bekannt und werden z. B. in Ullmanns Enzyklopädie der technischen Chemie, Band 1, 3. Auflage, 1951, S. 743 ff. beschrieben. Vorzugsweise wird für die kontinuierliche Variante des erfindungsgemäßen Verfahrens eine Rührkesselkaskade oder ein Rohrreaktor verwendet. Geeignete, gegebenenfalls druckfeste Reaktoren für die semikontinuierliche oder kontinuierliche Ausführung sind dem Fachmann bekannt und werden z. B. in Ullmanns Enzyklopädie der technischen Chemie, Band 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die erfindungsgemäßen Hydrocyanierungskatalysatoren lassen sich nach üblichen, dem Fachmann bekannten Verfahren vom Austrag der Hydrocyanierungsreaktion abtrennen und können im Allgemeinen erneut für die Hydrocyanierung eingesetzt werden.

Ein weiterer Gegenstand der Erfindung ist ein Verfahren zur Carbonylierung von Verbindungen, die wenigstens eine ethylenisch ungesättigte Doppelbindung enthalten, durch Umsetzung mit Kohlenmonoxid und wenigstens einer Verbindung mit einer nucleophilen Gruppe in Gegenwart eines Carbonylierungskatalysators, das dadurch gekennzeichnet ist, dass man als Carbonylierungskatalysator einen Katalysator auf Basis eines P-, As- oder Sb-haltigen Liganden der allgemeinen Formel I einsetzt.

Auch die erfindungsgemäßen Carbonylierungskatalysatoren umfassen Komplexe eines Metalls der VIII. Nebengruppe, bevorzugt Nickel, Cobalt, Eisen, Ruthenium, Rhodium und Palladium, insbesondere Palladium. Die Herstellung der Metallkomplexe kann wie bereits zuvor bei den Hydroformylierungskatalysatoren und Hydrocyanierungskatalysatoren beschrieben erfolgen. Gleiches gilt für die in situ-Herstellung der erfindungsgemäßen Carbonylierungskatalysatoren.

Geeignete Olefine für die Carbonylierung sind die allgemein zuvor als Einsatzstoffe für die Hydroformylierung und Hydrocyanierung genannten Olefine.

Vorzugsweise sind die Verbindungen mit einer nucleophilen Gruppe, ausgewählt unter Wasser, Alkoholen, Thiolen, Carbonsäureestern, primären und sekundären Aminen.

Eine bevorzugte Carbonylierungsreaktion ist die Überführung von Olefinen mit Kohlenmonoxid und Wasser zu Carbonsäuren (Hydrocarboxylierung). Dazu zählt insbesondere die Umsetzung von Ethylen mit Kohlenmonoxid und Wasser zu Propionsäure.

Ein weiterer Gegenstand der Erfindung ist die Verwendung der beschriebenen Katalysatoren, umfassend eine erfindungsgemäße P-, As- oder Sb-haltige Verbindung, zur Hydroformylierung, Hydrocyanierung und Carbonylierung.

Die Erfindung wird anhand der folgenden, nicht einschränkenden Beispiele näher erläutert.

### Beispiele

### I. Herstellung von Verbindungen I

### Beispiel 1:

### 1,8-Bis(diphenylphosphino)triptycen (= 1,8-Bis(diphenylphosphino)-9,10-dihydro-9,10-benzoanthracen) (Ligand A):

1.1 Herstellung von 1,8-Dichloranthracen
   25 g (91 mmol) 1,8-Dichloranthrachinon und 65 g (1 mol) Zn-Staub wurden in 1 1 einer 20 gew.-%igem wässrigen Ammoniaklösung suspendiert und 3 h zum Rückfluss erhitzt. Nach Abkühlen auf Raumtemperatur wurde der Feststoff über einen Büchnertrichter abgetrennt. Die wässrige Phase wurde dreimal mit je 150 ml Dichlormethan extrahiert; der Feststoff wurde unter Behandlung mit Ultraschall mehrfach mit Dichlormethan extrahiert. Die vereinigten organischen Phasen wurden über Magnesiumsulfat getrocknet und am Rotationsverdampfer vom Lösungsmittel befreit. Der feste weißlich-gelbe Rückstand wurde in 600 ml Propanol und 65 ml 12M Salzsäure suspendiert und 3 h zum Rückfluss erhitzt. Anschließend entfernte man das Lösungsmittel im Vakuum, nahm den Rückstand in Dichlormethan auf, wusch die Lösung mit gesättigter Natriumhydrogencarbonatlösung und trocknete über Magnesiumsulfat. Nach Entfernen des Lösungsmittels erhielt man 1,8-Dichloranthracen als gelben Feststoff, der aus Propanol umkristallisiert wurde.
   Ausbeute: 9,4 g (38 mmol) = 42 % d. Th.
   Schmelzpunkt: 155 °C (Literatur: 156-158 °C)
1.2 Herstellung von 1,8-Dichlortriptycen
   6,97 g (28 mmol) 1,8-Dichloranthracen wurden in 120 ml 1,2-Dichlorethan suspendiert und zum Rückfluss erhitzt. Zu der gelben Lösung gab man 3,2 g (31 mmol) n-Butylnitrit und tropfte hierzu eine Lösung von 4 g (29 mmol) Anthranilsäure in 25 ml 2-Ethoxyethylether. Nach einer halben Stunde destillierte man aus der schwarzen Lösung so lange das Lösungsmittel ab, bis die Temperatur am Destillationskopf 150 °C betrug. Nach Zugabe von 2 g (20 mmol) Maleinsäureanhydrid erhitzte man 3 min. zum Rückfluss, kühlte anschließend mit einem Eisbad und gab dann eine Lösung von 67 ml Methanol, 34,4 ml Wasser und 8 g Kaliumhydroxid zu. Der braune Feststoff wurde abfiltriert und solange mit Methanol:Wasser im Verhältnis 4:1 gewaschen, bis das Waschwasser farblos ablief.
   Der fleischfarbene Feststoff wurde in 2-Butanon gelöst, mit 200 mg Aktivkohle versetzt und diese Mischung eine Stunde zum Rückfluss erhitzt. Nach Abfiltrieren der Aktivkohle engte man die klare Lösung ein und ließ das Produkt im Kühlschrank auskristallisieren. Nach Zugabe von Methanol kristallisiert weiteres Produkt aus. Das Produkt kann aus 2-Butanon umkristallisiert werden.
   Ausbeute: 5,61 mg (17 mmol) = 62 %
   Schmelzpunkt: 297 °C (Literatur: 299,5-300 °C)
1.3 Herstellung von 1,8-Bis(diphenylphosphino)triptycen
   Alle Arbeitsschritte wurden unter Ausschluss von Sauerstoff und Wasser in einer Argonschutzgasatmosphäre durchgeführt.
   Zu einer auf -78 °C gekühlten Suspension von 82 mg (12 mmol) Lithiumpulver in 10 ml Tetrahydrofuran tropfte man eine Lösung von 485 mg (1,5 mmol) 9,10-Benzo-1,8-dichlor-9,10-dihydroanthracen in 20 ml Tetrahydrofuran im Lauf von einer Stunde. Die dabei erhaltene rote Suspension hielt man weitere 7 h bei -78 °C. Man filtrierte, gab 772 mg (3,5 mmol) Diphenylchlorphosphan zum Filtrat, entfernte die Kühlung, und rührte die gelbe Lösung 4 h bei Raumtemperatur. Nach Entfernen des gesamten Lösungsmittels im Vakuum wurde der verbleibende Feststoff mit Wasser versetzt und mit Dichlormethan extrahiert. Die Lösungsmittelphase wurde über Magnesiumsulfat getrocknet. Nach Entfernen des Dichlormethans im Vakuum kristallisierte man das Produkt aus Toluol um.
   Ausbeute: 392 mg (0,63 mmol) = 42 %
   Charakterisierung:
   ³¹P-NMR (CDCl₃, 121.495 MHz, 300K): δ = -14.7
   ¹H-NMR (C₆D₆, 300.132 MHz, 300K): δ = 5.20 (s, 1H, H10), 6.18 (s, 1H, H9), 6.62-7.34 (m, H-Ar)
   ¹³C-NMR (CDCl₃, 75.469 MHz, 300K): δ = 29.7 (C10), 59.0 (C9), 122.7-145.6 (C-Ar)

### Beispiel 2:

1,8-Bis(diphenylphosphino)-9,10-ethanoanthracen (Ligand B):
2.1 Die Herstellung von 1,8-Dichloranthracen erfolgte wie bei 1.1 beschrieben.
2.2 1,8-Dichloranthracen wurde nach der Vorschrift in JACS 94(9) (1972), S. 3080-3088 durch Diels-Alder-Reaktion mit Ethen zu 1,8-Dichlor-9,10-dihydro-9,10-ethanoanthracen umgesetzt.
2.3 Die Herstellung von 1,8-Bis(diphenylphosphino)-9,10-dihydro-9,10-ethanoanthracen erfolgte durch Lithiierung und Umsetzung mit Diphenylchlorphosphan, wie bei 1.3 beschrieben.

### II Untersuchung der Katalytischen Wirkung von Übergangsmetallkomplexen mit Liganden der Formel I

### Beispiel 3:

1,6 mg Biscarbonylrhodium(II)-2,4-pentandionat (Rh(CO)₂acac) und 19 mg 1,8-Bis(diphenylphosphino)triptycen (Ligand A, Ligand-Metall-Verhältnis 5:1 mol/mol) wurden in 5 ml Toluol gelöst und anschließend in einem Autoklaven bei 10 bar Synthesegasdruck (H₂/CO-Atmosphäre - Molverhältnis 1:1) 30 min. auf 100 °C erwärmt. Dann gab man 5 g Octen-1 zu, stellte einen Synthesegasdruck von 5 bar ein und hielt die Temperatur 4 h bei. Der Reaktionsaustrag wurde gaschromatographisch analysiert. Der Umsatz bezogen auf eingesetztes Octen-1 betrug 43 %. Die Selektivität hinsichtlich der Bildung von Nonanal betrug 92 %. Der Anteil an n-Nonanal betrug 99,2 %.

### Beispiel 4:

1,6 mg Rhodiumbiscarbonylacetylacetonat und 57 mg Ligand A (Ligand-Metall-Verhältnis = 15:1) wurden in 5 g Toluol gelöst und im Glasautoklav bei 10 bar CO/H₂ (1:1) in 30 min. auf 100 °C erwärmt. Anschließend wurden 5 g Octen-1 zugegeben und für 4 h ein Synthesegasdruck von 10 bar eingestellt. Eine Analyse des Reaktionsaustrags zeigte einen Umsatz von Octen-1 von 77 % mit einer Aldehydselektivität von 98 % und einer Linearität von 99 %. Der α-Anteil betrug 100 %.

### Beispiel 5:

1,6 mg Rhodiumbiscarbonylacetylacetonat, 76 mg Ligand A und 16 mg Triphenylphosphin (Triptyphos-Triphenylphosphin-Rhodium-Verhältnis = 20:10:1) wurden in 5 g Toluol gelöst und im Glasautoklav bei 10 bar CO/H₂ (1:1) in 30 min. auf 100 °C erwärmt. Anschließend wurden 5 g Octen-1 zugegeben, auf 120 °C erwärmt und für 4 h ein Synthesegasdruck von 10 bar eingestellt. Eine Analyse des Reaktionsaustrags zeigte einen Umsatz von Octen-1 von 97 % mit einer Aldehydselektivität von 92 % und einer Linearität von 99 %. Der α-Anteil betrug 100 %.

### Beispiel 6:

5,42 mg Rhodiumbiscarbonylacetylacetonat und 181,3 mg Ligand B wurden getrennt in je 5,5 g Diphenylether gelöst, zusammengegeben und bei 10 bar Synthesegasdruck (CO:H₂ = 1:1), 100 °C über 30 min. präformiert. Anschließend wurden mittels einer Schleuse 11 g 1-Octen (Reinheit: 97 % 1-Octen; Rest: interne Octene) zugegeben, und es wurde bei 100 °C und 10 bar 4 h hydroformyliert. Eine Analyse des Reaktionsaustrags mittels Gaschromatographie ergab einen Umsatz an 1-Octen von 98 %. Die Aldehydselektivität betrug 96 %, der n-Anteil 99 % und der α-Anteil (n- und iso-Aldehydanteil) 100 %.

Daraus wird ersichtlich, dass man unter Einsatz der neuen Katalysatoren mit hoher Linearität und sehr guter Aktivität bei gleichzeitig minimaler Isomerisierung zu innenständigen Olefinen endständige Olefine zu endständigen Aldehyden umsetzen kann.

## Patentansprüche

1. Verbindung der allgemeinen Formel I 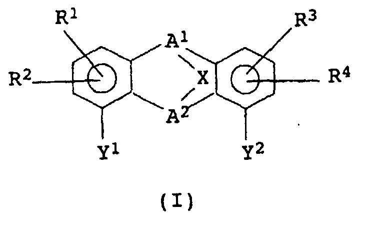 worin
A¹ und A² unabhängig voneinander für B, N, P oder CR⁵ stehen; wobei R⁵ für Wasserstoff, Alkyl, Cycloalkyl, Aryl oder Hetaryl steht und diese Reste wenigstens einen Substituenten aufweisen können, welcher ausgewählt ist aus COOR^{k}, COO⁻M⁺ SO₃R^{k}, SO₃⁻M⁺, NE³E⁴, NE³E⁴E⁵⁺X⁻, OR^{f}, SR^{f}, (CHR¹CH₂O)ₓR^{k} oder (CH₂CH₂N(E³))ₓR^{k}, worin R^{k}, E³, E⁴ und E⁵ jeweils gleiche oder verschiedene Reste, ausgewählt unter Wasserstoff, Alkyl, Cycloalkyl oder Aryl bedeuten; R¹ für Wasserstoff, Methyl oder Ethyl steht; M⁺ für ein Kation, wie Li⁺, Na⁺, K^{+,} NH₄⁺, steht; X⁻ für ein Anion, wie Cl- und Br- steht, und x für eine ganze Zahl von 1 bis 120 steht; oder R⁵ für einen, gegebenenfalls über eine Spacergruppe gebundenen, polymeren Träger steht,
X für O, S oder NR^{a} steht, wobei R^{a} für Wasserstoff, Alkyl, Cycloalkyl oder Aryl steht, oder X
für eine C₁- bis C₁₀-Alkylenbrücke steht, die eine, zwei, drei oder vier Doppelbindungen und/oder einen, zwei, drei oder vier Substituenten, die ausgewählt sind unter Alkyl, Alkoxy, Halogen, Nitro, Cyano, Carboxyl, Carboxylat, Cycloalkyl und Aryl, aufweisen kann, wobei der Arylsubstituent zusätzlich einen, zwei oder drei Substituenten, die ausgewählt sind unter Alkyl, Alkoxy, Halogen, SO₃H, Sulfonat, NE¹E², Alkylen-NE¹E², Trifluormethyl, Nitro, Alkoxycarbonyl oder Cyano, tragen kann, und/oder die Alkylenbrücke X durch ein, zwei oder drei nicht benachbarte, gegebenenfalls substituierte Heteroatome unterbrochen sein kann, und/oder die Alkylenbrücke X ein-, zwei- oder dreifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, SO₃H, Sulfonat, Trifluormethyl, Nitro, Cyano, Carboxyl, Alkoxycarbonyl, NE¹E² oder Alkylen-NE¹E², tragen können, wobei E¹ und E² gleich oder verschieden sein können und für Alkyl, Cycloalkyl oder Aryl stehen,
Y¹ und Y² unabhängig voneinander für mindestens ein Phosphor-, Arsen- oder Antimonatom aufweisende Reste stehen, worin das Phosphor-, Arsen- oder Antimonatom direkt oder über ein Sauerstoffatom an den Phenylring in Formel I gebunden ist,
R¹, R², R³ und R⁴ unabhängig voneinander für Wasserstoff, Alkyl, Cycloalkyl, Aryl, Alkoxy, Halogen, NE¹E², Alkylen-NE¹E², Trifluormethyl, Nitro, Alkoxycarbonyl oder Cyano stehen, wobei E¹ und E² gleich oder verschieden sind und für Alkyl, Cycloalkyl oder Aryl stehen.

2. Verbindung der allgemeinen Formel I, wie in Anspruch 1 definiert, worin A¹ und/oder A² für CR⁵ stehen, wobei R⁵ für einen, gegebenenfalls über eine Spacergruppe gebundenen, polymeren Träger steht.

3. Verbindung nach einem der vorhergehenden Ansprüche, worin Y¹ und Y² unabhängig voneinander für je einen Rest der Formeln PR⁶R⁷, P(OR⁶)R⁷, P(OR⁶)(OR⁷), OPR⁶R⁷, OP(OR⁶)R⁷ oder OP(OR⁶)(OR⁷) stehen, worin
R⁶ und R⁷ unabhängig voneinander für Alkyl, Cycloalkyl, Aryl oder Hetaryl stehen, welche gegebenenfalls einen, zwei oder drei Substituenten ausgewählt unter Alkyl, Cycloalkyl, Aryl, Alkoxy, Cycloalkyloxy, Aryloxy, Halogen, Trifluormethyl, Nitro, Cyano, Carboxyl, Carboxylat, Acyl, SO₃H, Sulfonat, NE¹E² und Alkylen-NE¹E² tragen, wobei E¹ und E² die zuvor genannten Bedeutungen haben, oder
R⁶ und R⁷ zusammen mit dem Phosphoratom und, falls vorhanden, dem/den Sauerstoffatom(en), an die sie gebunden sind, für einen 5- bis 8-gliedrigen Heterocyclus stehen, der gegebenenfalls zusätzlich ein-, zwei- oder dreifach mit Cycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, SO₃H, Sulfonat, NE¹E², Alkylen-NE¹E² Nitro, Cyano, Carboxyl und Carboxylat, tragen können und wobei der Heterocyclus gegebenenfalls zusätzlich ein oder zwei Heteroatom(e), die ausgewählt sind unter N, O und S, aufweisen kann.

4. Verbindung nach einem der vorhergehenden Ansprüche, worin
X für eine C₁- bis C₁₀-Alkylenbrücke steht, die eine, zwei, drei oder vier Doppelbindungen und/oder einen, zwei, drei oder vier Substituenten, die ausgewählt sind unter Alkyl, Alkoxy, Halogen, Nitro, Cyano, Carboxyl, Carboxylat, Cycloalkyl und Aryl, aufweisen kann, wobei der Arylsubstituent zusätzlich einen, zwei oder drei Substituenten, die ausgewählt sind unter Alkyl, Alkoxy, Halogen, SO₃H, Sulfonat, NE¹E², Alkylen-NE¹E², Trifluormethyl, Nitro, Alkoxycarbonyl oder Cyano, tragen kann, und/oder die Alkylenbrücke X durch ein, zwei oder drei nicht benachbarte, gegebenenfalls substituierte Heteroatome unterbrochen sein kann, und/oder die Alkylenbrücke X ein-, zwei- oder dreifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, SO₃H, Sulfonat, Trifluormethyl, Nitro, Cyano, Carboxyl, Alkoxycarbonyl, NE¹E² oder Alkylen-NE¹E², tragen können, wobei E¹ und E² gleich oder verschieden sein können und für Alkyl, Cycloalkyl oder Aryl stehen.

5. Verbindung nach Anspruch 4, wobei X ausgewählt ist unter Resten der Formeln II.1 bis II.10 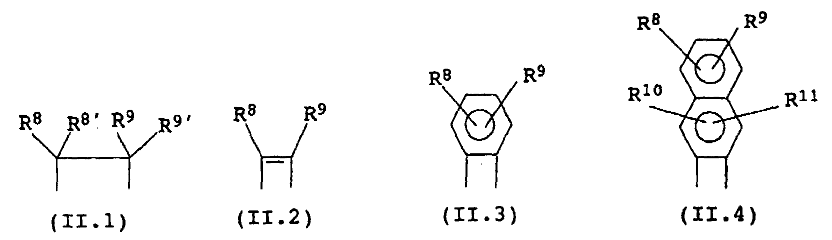 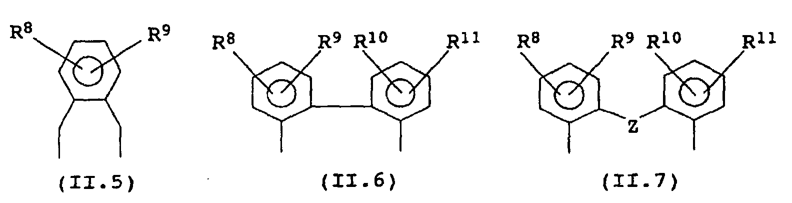 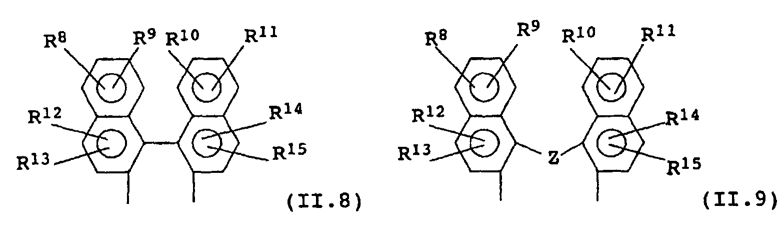 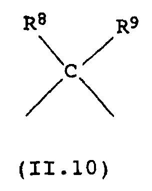 worin
Z für O, S oder NR¹⁶ steht, wobei R¹⁶ für Alkyl, Cycloalkyl oder Aryl steht,
oder Z für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei Substituenten, die ausgewählt sind unter Alkyl, Alkoxy, Halogen, SO₃H, Sulfonat, NE¹E², Alkylen-NE¹E², Trifluormethyl, Nitro, Alkoxycarbonyl oder Cyano, tragen kann,
oder Z für eine C₂- bis C₃-Alkylenbrücke steht, die durch O, S oder NR¹⁶ unterbrochen ist,
R⁸, R^{8'}, R⁹, R^{9'}, R¹⁰, R¹¹, R¹², R¹³, R¹⁴ und R¹⁵ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen.

6. Verbindung nach einem der Ansprüche 3 bis 5, wobei R⁶ und R⁷ zusammen mit dem Phosphoratom und, falls vorhanden, dem/den Sauerstoffatom(en), an die sie gebunden sind, für einen 5-bis 8-gliedrigen Heterocyclus stehen, wobei R⁶ und R⁷ gemeinsam für einen Rest stehen, der ausgewählt ist unter den Resten II.5 bis II.9, wie in Anspruch 5 definiert.

7. Verbindung nach einem der vorhergehenden Ansprüche, die ausgewählt ist unter Verbindungen der Formeln I.i bis I.v 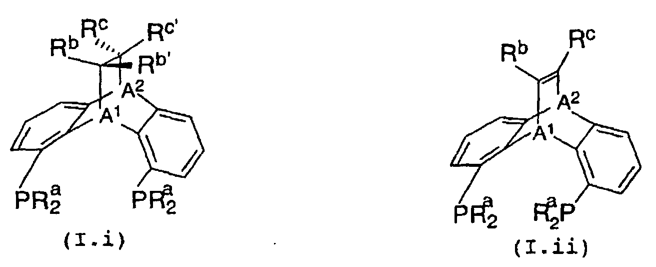 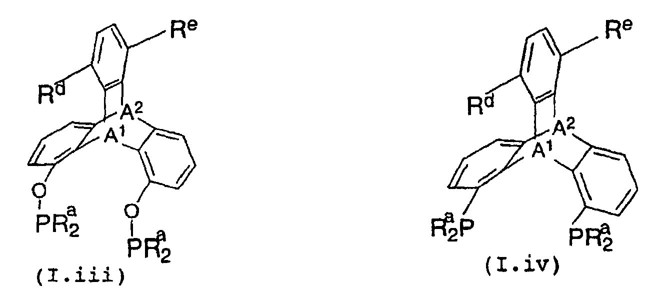 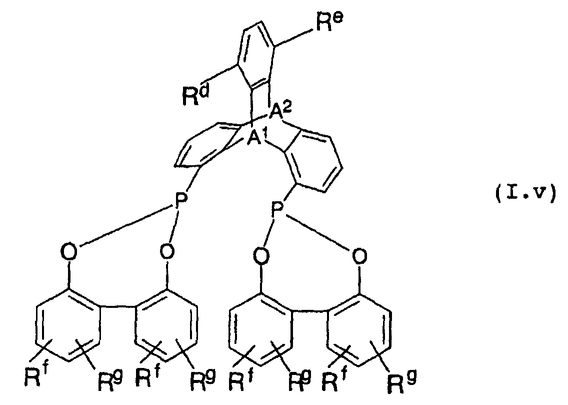 worin
R^{a} ausgewählt ist unter C₁-C₆-Alkyl, C₁-C₆-Alkoxy, C₅-C₇-Cycloalkoxy, Phenyl, Phenoxy und Pentafluorphenyl, wobei die Phenyl- und Phenoxyreste gegebenenfalls einen Substituenten, ausgewählt unter Carboxyl, Carboxylat, -SO₃H und Sulfonat aufweisen können,
R^{b}, R^{b'}, R^{c} und R^{c'} unabhängig voneinander ausgewählt sind unter Wasserstoff, C₁-C₆-Alkyl, C₁-C₆-Alkoxy, C₁-C₆-Alkoxycarbonyl und Aryl,
R^{d} und R^{e} unabhängig voneinander ausgewählt sind unter Wasserstoff und C₁-C₆-Alkyl,
R^{f} und R^{g} unabhängig voneinander ausgewählt sind unter Wasserstoff, C₁-C₆-Alkyl und C₁-C₆-Alkoxy, und
A¹ und A² unabhängig voneinander für N oder CR⁵ stehen, wobei R⁵ für Wasserstoff oder C₁-C₈-Alkyl steht.

8. Verfahren zur Herstellung von Verbindungen der allgemeinen Formel I, wobei X für einen Rest der Formeln II.1, II.2, II.3 oder II.4 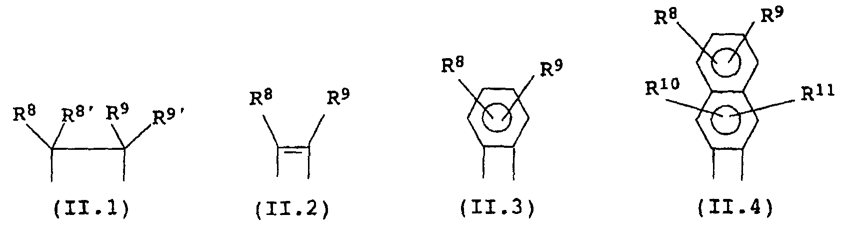 steht, worin
R⁸, R^{8'}, R⁹, R^{9'}, R¹⁰ und R¹¹ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl, Carboxyl oder Cyano stehen,
durch Umsetzung einer Verbindung der allgemeinen Formel I.1 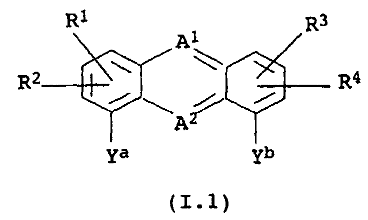 worin
A¹ und A² unabhängig voneinander für B, N, P oder CR⁵ stehen, wobei R⁵ für Wasserstoff, Alkyl, Cycloalkyl, Aryl, Hetaryl oder einen, gegebenenfalls über eine Spacergruppe gebundenen, polymeren Träger steht,
Y^{a} und Y^{b} unabhängig voneinander für Reste Y¹ oder Y², wie in Anspruch 1 definiert, stehen,
oder Y^{a} und Y^{b} unabhängig voneinander für Halogen, OH, OC(O)CF₃ oder SO₃Me mit Me = Wasserstoff, Li, Na oder K, stehen, wobei Y^{a} und/oder Y^{b} auch für Wasserstoff stehen kann, wenn jeweils einer der Reste R² und/oder R⁴ eine Alkoxygruppe bedeutet, die sich in der ortho-Position von Y^{a} und/oder Y^{b} befindet,
R¹, R², R³ und R⁴ unabhängig voneinander für Wasserstoff, Alkyl, Cycloalkyl, Aryl, Alkoxy, Halogen, SO₃H, Sulfonat, NE¹E², Alkylen-NE¹E², Trifluormethyl, Nitro, Alkoxycarbonyl oder Cyano stehen,
mit einer Verbindung, ausgewählt unter Verbindungen der Formeln II.a, II.b, II.c oder II.d 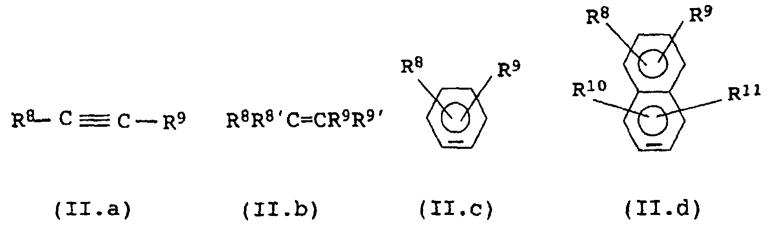 oder einer zur Ausbildung einer Verbindung der Formeln II.c oder II.d geeigneten Vorstufe, in einer [4+2]-Cycloaddition (Diels-Alder-Reaktion) und
Funktionalisierung von Resten Y^{a} und/oder Y^{b} zu Resten Y¹ und/oder Y².

9. Katalysator, umfassend wenigstens einen Komplex eines Metalls der VIII. Nebengruppe mit mindestens einem Liganden, der ausgewählt ist unter Verbindungen der allgemeinen Formel I, wie in einem der Ansprüche 1 bis 7 definiert.

10. Katalysator nach Anspruch 9, **dadurch gekennzeichnet, dass** das Metall der VIII. Nebengruppe ausgewählt ist unter Cobalt, Ruthenium, Iridium, Rhodium, Nickel, Palladium und Platin.

11. Katalysator nach einem der Ansprüche 9 oder 10, der zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit und Phosphitliganden aufweist.

12. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, **dadurch gekennzeichnet, dass** man als Hydroformylierungskatalysator einen Katalysator nach einem der Ansprüche 9 bis 11 einsetzt.

13. Verfahren zur Hydrocyanierung von Verbindungen, die wenigstens eine ethylenisch ungesättigte Doppelbindung enthalten, durch Umsetzung mit Cyanwasserstoff in Gegenwart eines Hydrocyanierungskatalysators, **dadurch gekennzeichnet, dass** man als Hydrocyanierungskatalysator einen Katalysator nach einem der Ansprüche 9 bis 11 einsetzt.

14. Verfahren nach einem der Ansprüche 12 oder 13, **dadurch gekennzeichnet, dass** der Hydroformylierungskatalysator oder der Hydrocyanierungskatalysator in situ hergestellt wird, wobei man mindestens eine Verbindung der allgemeinen Formel I, wie in den Ansprüchen 1 bis 7 definiert, eine Verbindung oder einen Komplex eines Metalls der VIII. Nebengruppe und gegebenenfalls ein Aktivierungsmittel in einem inerten Lösungsmittel unter den Hydroformylierungsbedingungen oder Hydrocyanierungsbedingungen zur Reaktion bringt.

15. Verfahren zur Carbonylierung von Verbindungen, die wenigstens eine ethylenisch ungesättigte Doppelbindung enthalten, durch Umsetzung mit Kohlenmonoxid und wenigstens einer Verbindung mit einer nucleophilen Gruppe in Gegenwart eines Carbonylierungskatalysators, **dadurch gekennzeichnet, dass** man als Carbonylierungskatalysator einen Katalysator nach einem der Ansprüche 9 bis 11 einsetzt.

16. Verwendung von Katalysatoren, umfassend wenigstens einen Komplex eines Metalls der VIII. Nebengruppe mit mindestens einem Liganden, der ausgewählt ist unter Verbindungen der allgemeinen Formel I, wie in einem der Ansprüche 1 bis 7 definiert, zur Hydroformylierung, Hydrocyanierung und Carbonylierung.

## Claims

1. A compound of the formula I 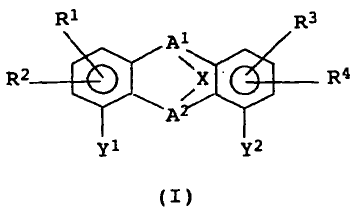 where
A¹ and A² are each, independently of one another, B, N, P or CR⁵, where R⁵ is hydrogen, alkyl, cycloalkyl, aryl or heteroaryl and these radicals may be substituted by one or more substituents selected from COOR^{k}, COO⁻M⁺, SO₃R^{k}, SO₃⁻M⁺, NE³E⁴, NE³E⁴E⁵⁺X⁻, OR^{f}, SR^{f}, (CHR¹CH₂O)ₓR^{k} and (CH₂CH₂N(E³))ₓR^{k}, where R^{k}, E³, E⁴ and E⁵ are each identical or different radicals selected from among hydrogen, alkyl, cycloalkyl or aryl; R¹ is hydrogen, methyl or ethyl; M⁺ is a cation, such as Li⁺, Na⁺, K⁺, NH₄⁺; X⁻ is an anion, such as Cl⁻ and Br-, and x is an integer from 1 to 120; or R⁵ is a polymeric support which may be bound via a spacer group,
X is O, S or NR^{a}, where R^{a} is hydrogen, alkyl, cycloalkyl or aryl, or X
is a C₁-C₁₀-alkylene bridge which may contain one, two, three or four double bonds and/or may bear one, two, three or four substituents selected from among alkyl, alkoxy, halogen, nitro, cyano, carboxyl, carboxylate, cycloalkyl and aryl, where the aryl substituent may additionally bear one, two or three substituents selected from among alkyl, alkoxy, halogen, SO₃H, sulfonate, NE¹E², alkylene-NE¹E², trifluoromethyl, nitro, alkoxycarbonyl or cyano, and/or the alkylene bridge X may be interrupted by one, two or three nonadjacent, substituted or unsubstituted heteroatoms, and/or the alkylene bridge X may be fused with one, two or three aryl and/or hetaryl rings, where the fused-on aryl and hetaryl groups may each bear one, two or three substituents selected from among alkyl, cycloalkyl, aryl, alkoxy, cycloalkoxy, aryloxy, acyl, halogen, SO₃H, sulfonate, trifluoromethyl, nitro, cyano, carboxyl, alkoxycarbonyl, NE¹E² and alkylene-NE¹E², where E¹ and E² may be identical or different and are each alkyl, cycloalkyl or aryl,
Y¹ and Y² are, independently of one another, radicals containing at least one phosphorus, arsenic or antimony atom, where the phosphorus, arsenic or antimony atom is bound directly or via an oxygen atom to the phenyl ring in formula I,
R¹, R², R³ and R⁴ are each, independently of one another, hydrogen, alkyl, cycloalkyl, aryl, alkoxy, halogen, NE¹E², alkylene-NE¹E², trifluoromethyl, nitro, alkoxycarbonyl or cyano, where E¹ and E² are identical or different and are each alkyl, cycloalkyl or aryl.

2. A compound of the formula I as defined in claim 1 in which A¹ and/or A² are/is CR⁵, where R⁵ is a polymeric support which may be bound via a spacer group.

3. A compound as claimed in either of the preceding claims, wherein Y¹ and Y² are each, independently of one another, a radical of the formula PR⁶R⁷, P(OR⁶)R⁷, P(OR⁶)(OR⁷), OPR⁶R⁷, OP(OR⁶)R⁷ or OP(OR⁶)(OR⁷), where
R⁶ and R⁷ are each, independently of one another, alkyl, cycloalkyl, aryl or heteroaryl which may bear one, two or three substituents selected from among alkyl, cycloalkyl, aryl, alkoxy, cycloalkyloxy, aryloxy, halogen, trifluoromethyl, nitro, cyano, carboxyl, carboxylate, acyl, SO₃H, sulfonate, NE¹E² and alkylene-NE¹E², where E¹ end E² are as defined above, or
R⁶ and R⁷ together with the phosphorus atom and, if present, the oxygen atom(s) to which they are bound form a 5- to 8-membered heterocycle which may additionally be fused with one, two or three cycloalkyl, aryl and/or heteroaryl rings, where the fused-on groups may each bear, independently of one another, one, two, three or four substituents selected from among alkyl, alkoxy, halogen, SO₃H, sulfonate, NE¹E², alkylene-NE¹E², nitro, cyano, carboxyl and carboxylate and where the heterocycle may additionally contain one or two heteroatoms(s) selected from among N, O and S.

4. A compound as claimed in any of the preceding claims, wherein
X is a C₁-C₁₀-alkylene bridge which may contain one, two, three or four double bonds and/or may bear one, two, three or four substituents selected from among alkyl, alkoxy, halogen, nitro, cyano, carboxyl, carboxylate, cycloalkyl and aryl, where the aryl substituent may additionally bear one, two or three substituents selected from among alkyl, alkoxy, halogen, SO₃H, sulfonate, NE¹E², alkylene-NE¹E², trifluoromethyl, nitro, alkoxycarbonyl and cyano, and/or the alkylene bridge X may be interrupted by one, two or three nonadjacent, substituted or unsubstituted heteroatoms, and/or the alkylene bridge X may be fused with one, two or three aryl and/or heteroaryl rings, where the fused-on aryl and heteroaryl groups may each bear one, two or three substituents selected from among alkyl, cycloalkyl, aryl, alkoxy, cycloalkoxy, aryloxy, acyl, halogen, SO₃H, sulfonate, trifluoromethyl, nitro, cyano, carboxyl, alkoxycarbonyl, NE¹E² and alkylene-NE¹E², where E¹ and E² may be identical or different and are each alkyl, cycloalkyl or aryl.

5. A compound as claimed in claim 4, wherein X is selected from among radicals of the formulae II.1 to II.10 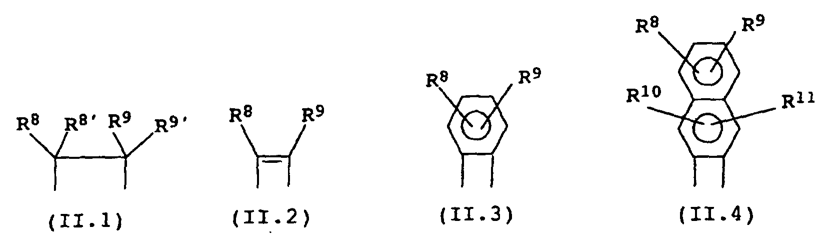 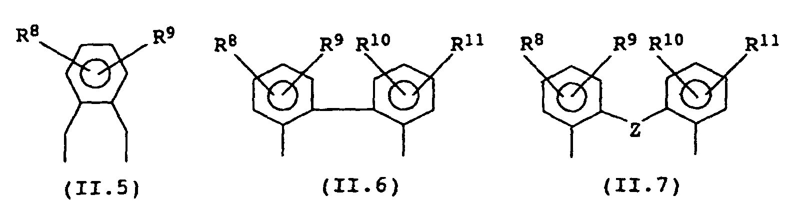 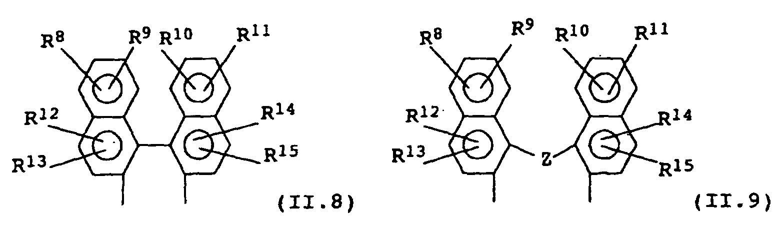 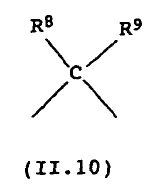 where
Z is O, S or NR¹⁶, where R¹⁶ is alkyl, cycloalkyl or aryl,
or Z is a C₁-C₃-alkylene bridge which may contain a double bond and/or bear an alkyl, cycloalkyl or aryl substituent, where the aryl substituent may bear one, two or three substituents selected from among alkyl, alkoxy, halogen, SO₃H, sulfonate, NE¹E², alkylene-NE¹E², trifluoromethyl, nitro, alkoxycarbonyl and cyano,
or Z is a C₂-C₃-alkylene bridge which is interrupted by O, S or NR¹⁶,
R⁸, R^{8'}, R⁹, R^{9'}, R¹⁰, R¹¹, R¹², R¹³, R¹⁴ and R¹⁵ are each, independently of one another, hydrogen, alkyl, cycloalkyl, aryl, alkoxy, halogen, SO₃H, sulfonate, NE¹E², alkylene-NE¹E², trifluoromethyl, nitro, alkoxycarbonyl, carboxyl or cyano.

6. A compound as claimed in any of claims 3 to 5, wherein R⁶ and R⁷ together with the phosphorus atom and, if present, the oxygen atom(s) to which they are bound form a 5- to 8-membered heterocycle, where R⁶ and R⁷ are together a radical selected from among the radicals II.5 to II.9 as defined in claim 5.

7. A compound as claimed in any of the preceding claims which is selected from among compounds of the formulae I.i to I.v 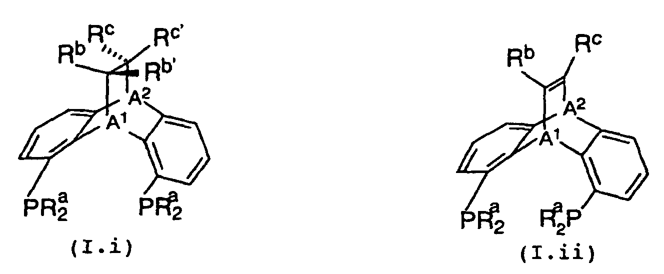 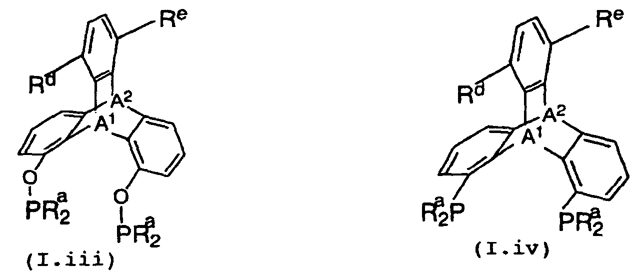 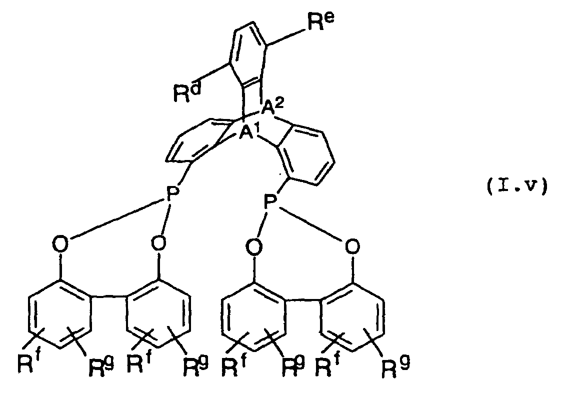 where
R^{a} is selected from among C₁-C₆-alkyl, C₁-C₆-alkoxy, C₅-C₇-cycloalkoxy, phenyl, phenoxy and pentafluorophenyl, where the phenyl- and phenoxy radicals may bear a substituent selected from among carboxyl, carboxylate, - SO₃H and sulfonate,
R^{b}, R^{b'}, R^{c} and R^{c'} are selected independently from among hydrogen, C₁-C₆-alkyl, C₁-C₆-alkoxy, C₁-C₆-alkoxycarbonyl and aryl,
R^{d} and R^{e} are selected independently from among hydrogen and C₁-C₆-alkyl,
R^{f} and R^{g} are selected independently from among hydrogen, C₁-C₆-alkyl and C₁-C₆-alkoxy, and
A¹ and A² are each, independently of one another, N or CR⁵, where R⁵ is hydrogen or C₁-C₈-alkyl.

8. A process for preparing a compound of the formula I in which X is a radical of the formulae II.1, II.2, II.3 or II.4 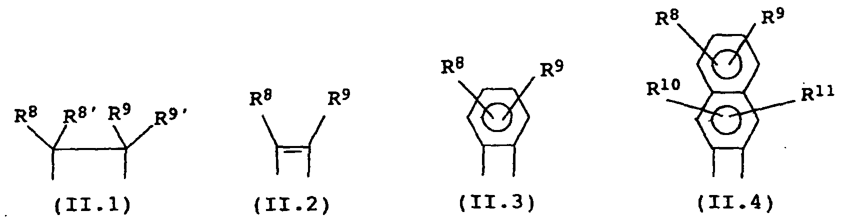 where
R⁸, R^{8'}, R⁹, R^{9'}, R¹⁰ and R¹¹ are each, independently of one another, hydrogen, alkyl, cycloalkyl, aryl, alkoxy, halogen, SO₃H, sulfonate, NE¹E², alkylene-NE¹E², trifluoromethyl, nitro, alkoxycarbonyl, carboxyl or cyano,
by reacting a compound of the formula I.1 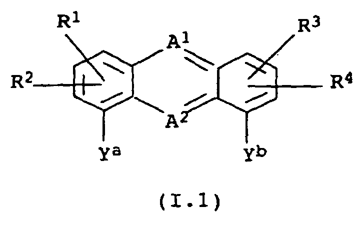 where
A¹ and A² are each, independently of one another, B, N, P or CR⁵, where R⁵ is hydrogen, alkyl, cycloalkyl, aryl, heteroaryl or a polymeric support which may be bound via a spacer group,
Y^{a} and Y^{b} are each, independently of one another, radicals Y¹ or Y² as defined in claim 1,
or Y^{a} and Y^{b} are each, independently of one another, halogen, OH, OC(O)CF₃ or SO₃Me where Me = hydrogen, Li, Na or K, where Y^{a} and/or Y^{b} can also be hydrogen if in each case one of the radicals R² and/or R⁴ is an alkoxy group which is located in the ortho position of Y^{a} and/or Y^{b},
R¹, R², R³ and R⁴ are each, independently of one another, hydrogen, alkyl, cycloalkyl, aryl, alkoxy, halogen, SO₃H, sulfonate, NE¹E², alkylene-NE¹E², trifluoromethyl, nitro, alkoxycarbonyl or cyano,
with a compound selected from among compounds of the formulae II.a, II.b, II.c or II.d 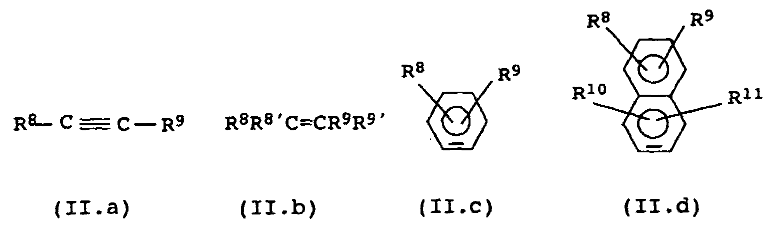 or a precursor of a compound of the formula II.c or II.d in a [4+2]-cycloaddition (Diels-Alder reaction) and
functionalizing radicals Y^{a} and/or Y^{b} to form radicals Y¹ and/or Y².

9. A catalyst comprising at least one complex of a metal of transition group VIII with at least one ligand selected from among compounds of the formula I as defined in any of claims 1 to 7.

10. A catalyst as claimed in claim 9, wherein the metal of transition group VIII is selected from among cobalt, ruthenium, iridium, rhodium, nickel, palladium and platinum.

11. A catalyst as claimed in claim 9 or 10 which further comprises at least one additional ligand selected from among halides, amines, carboxylates, acetylacetonate, arylsulfonates and alkylsulfonates, hydride, CO, olefins, dienes, cycloolefins, nitriles, N-containing heterocycles, aromatics and heteroaromatics, ethers, PF₃, phospholes, phosphabenzenes and monodentate, bidentate and polydentate phosphine, phosphinite, phosphonite, phosphoramidite and phosphite ligands.

12. A process for the hydroformylation of compounds containing at least one ethylenically unsaturated double bond by reaction with carbon monoxide and hydrogen in the presence of a catalyst as claimed in any of claims 9 to 11 as hydroformylation catalyst.

13. A process for the hydrocyanation of compounds containing at least one ethylenically unsaturated double bond by reaction with hydrogen cyanide in the presence of a catalyst as claimed in any of claims 9 to 11 as hydrocyanation catalyst.

14. A process as claimed in claim 12 or 13, wherein the hydroformylation catalyst or the hydrocyanation catalyst is prepared in situ by reacting at least one compound of the formula I as defined in any of claims 1 to 7, a compound or complex of a metal of transition group VIII and, if desired, an activating agent in an inert solvent under the hydroformylation conditions or hydrocyanation conditions.

15. A process for the carbonylation of compounds which contain at least one ethylenically unsaturated double bond by reaction with carbon monoxide and at least one compound containing a nucleophilic group in the presence of a catalyst as claimed in any of claims 9 to 11 as carbonylation catalyst.

16. The use of a catalyst comprising at least one complex of a metal of transition group VIII with at least one ligand selected from among compounds of the formula I as defined in any of claims 1 to 7 for hydroformylation, hydrocyanation and carbonylation.

## Revendications

1. Composé de la formule générale I : 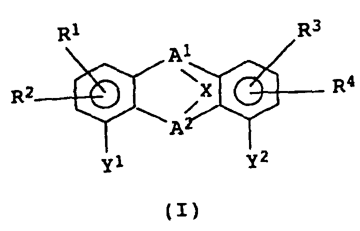 dans laquelle :
A¹ et A² représentent indépendamment l'un de l'autre, B, N, P ou CR⁵, où R⁵ représente hydrogène, alkyle, cycloalkyle, aryle ou hétéroaryle et ces restes peuvent présentent au moins un substituant, qui sont choisis parmi COOR^{k}, COO⁻M⁺, SO₃R^{k}, SO₃⁻M⁺, NE³E⁴, NE³E⁴E⁵⁺X⁻, OR^{f}, SR^{f}, (CHR¹CH₂O)ₓR^{k} ou (CH₂CH₂N(E³))ₓR^{K}, où R^{k}, E³, E⁴ et E⁵ représentent chaque fois, des restes identiques ou différentes, choisis parmi hydrogène, alkyle, cycloalkyle ou aryle ; R¹ représente hydrogène, méthyle ou éthyle ; M⁺ représente un cation comme Li⁺, Na⁺, K⁺, NH₄⁺ ; X⁻ représente un anion comme Cl⁻ et Br⁻, et x représente un nombre entier allant de 1 à 120, ou R⁵ représente un support polymère, le cas échéant relié par un espaceur ;
X représente O, S ou NR^{a}, où R^{a} représente hydrogène, alkyle, cyloalkyle ou aryle, ou X représente un pont alkylène en C₁ à C₁₀, qui peut présenter une, deux, trois ou quatre doubles liaisons et/ou un, deux, trois ou quatre substituants, qui sont choisis parmi alkyle, alcoxy, halogène, nitro, cyano, carboxyle, carboxylate, cycloalkyle et aryle, où le substituant aryle peut en outre, porter un, deux ou trois substituants, qui sont choisis parmi alkyle, alcoxy, halogène, SO₃H, sulfonate, NE¹E², alkylène-NE¹E², trifluorométhyle, nitro, alcoxycarbonyle ou cyano, et/ou le pont alkylène X peut être interrompu par un, deux ou trois hétéroatomes le cas échéant substitués, non voisins, et/ou le pont alkylène X peut être condensé une, deux ou trois fois, avec aryle et/ou hétéroaryle, où les radicaux aryle et hétéroaryle condensés peuvent chacun porter un, deux ou trois substituants qui sont choisis parmi alkyle, cycloalkyle, aryle, alcoxy, cycloalcoxy, aryloxy, acyle, halogène, SO₃H, sulfonate, trifluorométhyle, nitro, cyano, carboxyle, alcoxycarbonyle, NE¹E² ou alkylène-NE¹E², où E¹ et E² peuvent être identiques ou différents et représentent alkyle, cycloalkyle ou aryle ;
Y¹ et Y² représentent indépendamment l'un de l'autre, au moins un reste présentant un atome de phosphore, d'arsenic ou d'antimoine, où l'atome de phosphore, d'arsenic ou d'antimoine, est relié directement ou par un atome d'oxygène sur le cycle phényle de la formule I ;
R¹, R², R³ et R⁴ représentent indépendamment l'un de l'autre, hydrogène, alkyle, cycloalkyle, aryle, alcoxy, halogène, NE¹E², alkylène-NE¹E², trifluorométhyle, nitro, alcoxycarbonyle ou cyano, où E¹ et E² sont identiques ou différents et représentent alkyle, cycloalkyle ou aryle.

2. Composé de la formule générale I, comme défini à la revendication 1, où A¹ et/ou A² représentent CR⁵, où R⁵ représente un support polymère, le cas échéant relié par un espaceur.

3. Composé selon l'une quelconque des revendications précédentes, où Y¹ et Y² représentent indépendamment l'un de l'autre, chaque fois un reste de la formule PR⁶R⁷, P(OR⁶)R⁷, P(OR⁶) (OR⁷), OPR⁶R⁷, OP(OR⁶)R⁷ ou OP(OR⁶) (OR⁷), où
R⁶ et R⁷ représentent indépendamment l'un de l'autre, alkyle, cycloalkyle, aryle ou hétéroaryle, qui peuvent le cas échéant, porter un, deux ou trois substituants choisis parmi alkyle, cycloalkyle, aryle, alcoxy, cycloalcoxy, aryloxy, halogène, trifluorométhyle, nitro, cyano, carboxyle, carboxylate, acyle, SO₃H, sulfonate, NE¹E² et alkylène-NE¹E², où E¹ et E² ont la signification indiquée ci-dessus, ou
R⁶ et R⁷ avec l'atome de phosphore, et si présent, le ou les atomes d'oxygène, sur lesquels ils sont liés, représentent un hétérocycle à 5 à 8 membres, qui peut être condensé, le cas échéant, en outre, une, deux ou trois fois, avec un cycloalkyle, aryle et/ou hétéroaryle, où les radicaux condensés peuvent porter chaque fois, un, deux, trois ou quatre substituants, choisis parmi alkyle, cycloalkyle, alcoxy, halogène, SO₃H, sulfonate, NE¹E², alkylène-NE¹E², nitro, cyano, carboxyle et carboxylate, et où l'hétérocycle peut présenter en outre, le cas échéant un ou deux hétéroatomes, qui sont choisis parmi N, O et S.

4. Composé selon l'une quelconque des revendications précédentes, où X représente un pont alkylène en C₁ à C₁₀, qui peut présenter une, deux, trois ou quatre doubles liaisons et/ou un, deux, trois ou quatre substituants, qui sont choisis parmi alkyle, alcoxy, halogène, nitro, cyano, carboxyle, carboxylate, cycloalkyle et aryle, où le substituant aryle peut en outre, porter un, deux ou trois substituants, qui sont choisis parmi alkyle, alcoxy, halogène, SO₃H, sulfonate, NE¹E², alkylène-NE¹E², trifluorométhyle, nitro, alcoxycarbonyle ou cyano, et/ou le pont alkylène X peut être interrompu par un, deux ou trois hétéroatomes le cas échéant substitués, non voisins, et/ou le pont alkylène X peut être condensé une, deux ou trois fois, avec aryle et/ou hétéroaryle, où les radicaux aryle et hétéroaryle condensés peuvent chacun porter un, deux ou trois substituants qui sont choisis parmi alkyle, cycloalkyle, aryle, alcoxy, cycloalcoxy, aryloxy, acyle, halogène, SO₃H, sulfonate, trifluorométhyle, nitro, cyano, carboxyle, alcoxycarbonyle, NE¹E² ou alkylène-NE¹E², où E¹ et E² peuvent être identiques ou différents et représentent alkyle, cycloalkyle ou aryle.

5. Composé selon la revendication 4, où X est choisi parmi les restes des formules II.1 à II.10 : 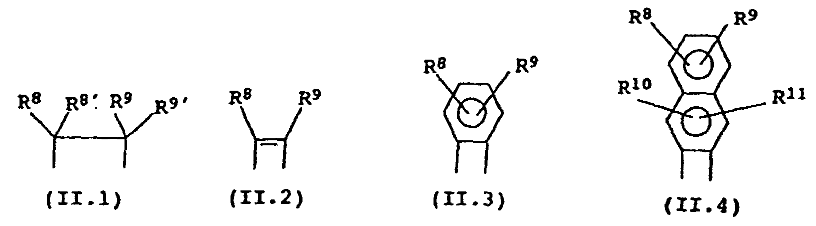 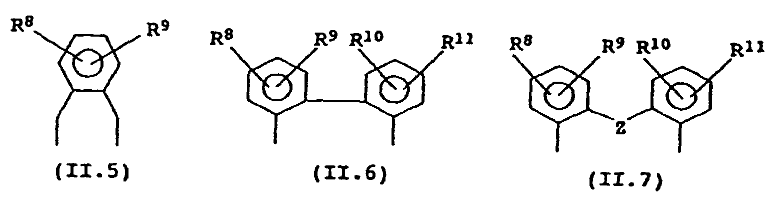 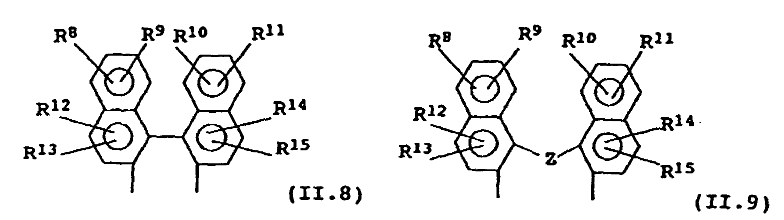 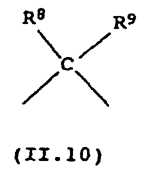 où
Z représente O, S ou NR¹⁶, où
R¹⁶ représente alkyle, cycloalkyle ou aryle, ou
Z représente un pont alkylène en C₁ à C₃, qui peut présenter une double liaison et/ou un substituant alkyle, cycloalkyle ou aryle, où le substituant aryle peut porter un, deux ou trois substituants, qui sont choisis parmi alkyle, alcoxy, halogène, SO₃H, sulfonate, NE¹E², alkylène-NE¹E², trifluorométhyle, nitro, alcoxycarbonyle ou cyano, ou
Z représente un pont alkylène en C₂ à C₃, qui peut être interrompu par O, S ou NR¹⁶ ;
R⁸, R^{8'}, R⁹, R^{9'}, R¹⁰, R¹¹, R¹², R¹³, R¹⁴ et R¹⁵ représentent indépendamment l'un de l'autre, hydrogène, alkyle, cycloalkyle, aryle, alcoxy, halogène, SO₃H, sulfonate, NE¹E², alkylène-NE¹E², trifluorométhyle, nitro, alcoxycarbonyle, carboxyle ou cyano.

6. Composé selon l'une quelconque des revendications 3 à 5, où R⁶ et R⁷ avec l'atome de phosphore, et si présent, le ou les atomes d'oxygène, sur lesquels ils sont liés, représentent un hétérocycle à 5 à 8 membres, ou R⁶ et R⁷ représentent ensemble, un reste qui est choisi parmi les restes II.5 à II.9, comme défini à la revendication 5.

7. Composé selon l'une quelconque des revendications précédentes, qui est choisi parmi les composés des formules I.i à I.v : 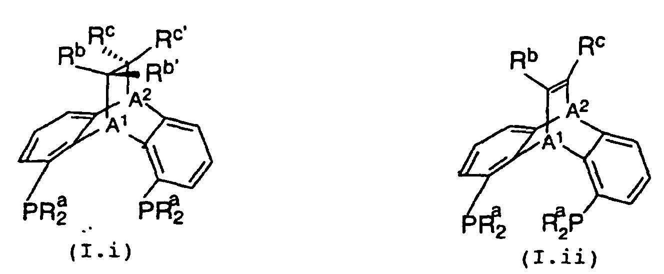 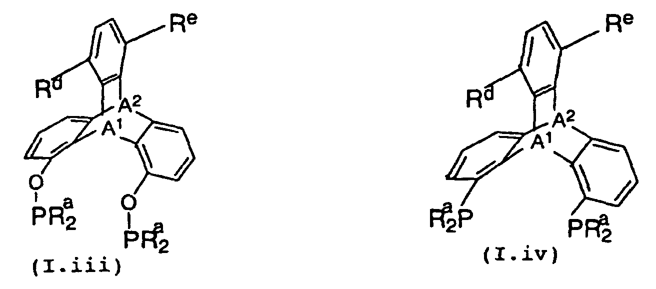 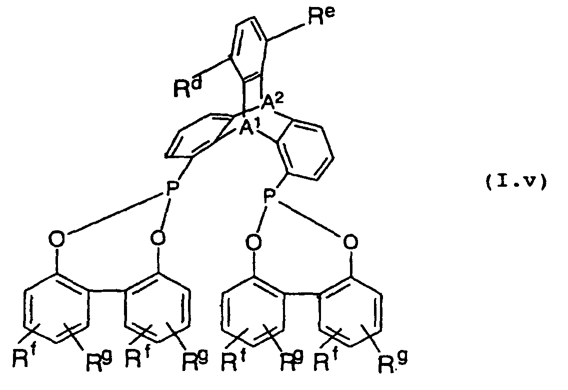 où
R^{a} est choisi parmi alkyle en C₁-C₆, alcoxy en C₁-C₆, cycloalkyle en C₅-C₇, phényle, phénoxy et pentafluorophényle, où les restes phényle et phénoxy peuvent le cas échéant, présenter un substituant choisi parmi carboxyle, carboxylate, -SO₃H et sulfonate,
R^{b}, R^{b'}, R^{c} et R^{c'} sont choisis indépendamment l'un de l'autre, parmi hydrogène, alkyle en C₁-C₆, alcoxy en C₁-C₆, alcoxycarbonyle en C₁-C₆, et aryle,
R^{d} et R^{d'} sont choisis indépendamment l'un de l'autre, parmi hydrogène et alkyle en C₁-C₆,
R^{f} et R^{g} sont choisis indépendamment l'un de l'autre, parmi hydrogène, alkyle en C₁-C₆ et alcoxy en C₁-C₆, et
A¹ et A² représentent indépendamment l'un de l'autre, N ou CR⁵, où R⁵ représente hydrogène et alkyle en C₁-C₈.

8. Procédé de préparation des composés de la formule générale I, où X représente un reste des formules II.1, II.2, II.3 ou II.4 : 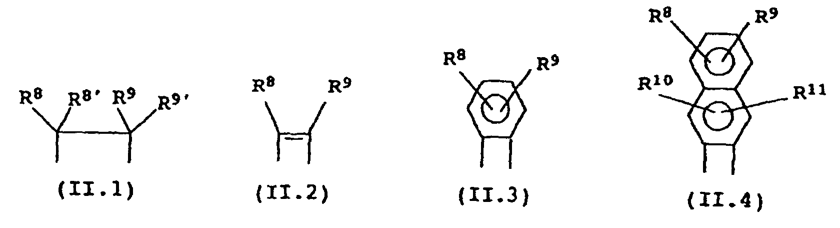 où
R⁸, R^{8'}, R⁹, R^{9'}, R¹⁰ et R¹¹, représentent indépendamment l'un de l'autre, hydrogène, alkyle, cycloalkyle, aryle, alcoxy, halogène, SO₃H, sulfonate, NE¹E², alkylène-NE¹E², trifluorométhyle, nitro, alcoxycarbonyle, carboxyle ou cyano,
par réaction d'un composé de la formule générale I.1 : 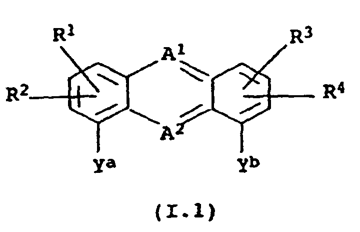 où
A¹ et A² représentent indépendamment l'un de l'autre, B, N, P ou CR⁵, où R⁵ représente hydrogène, alkyle, cycloalkyle, aryle ou hétéroaryle ou un support polymère le cas échéant relié par un espaceur,
Y^{a} et Y^{b} représentent indépendamment l'un de l'autre, les restes Y¹ ou Y², comme défini à la revendication 1, ou
Y^{a} et Y^{b} représentent indépendamment l'un de l'autre, halogène, OH, OC(O)CF₃ ou SO₃Me avec Me = hydrogène, Li, Na ou K, où Y^{a} et/ou Y^{b} peut représenter également, hydrogène, lorsque chaque fois, un des restes R² et/ou R⁴ représente un radical alcoxy, qui se trouve en position ortho de Y^{a} et/ou Y^{b},
R¹, R², R³ et R⁴ représentent indépendamment l'un de l'autre, hydrogène, alkyle, cycloalkyle, aryle, alcoxy, halogène, SO₃H, sulfonate, NE¹E², alkylène-NE¹E², trifluorométhyle, nitro, alcoxycarbonyle ou cyano,
avec un composé, choisi parmi les composés des formules II.a, II.b, II.c ou II.d : 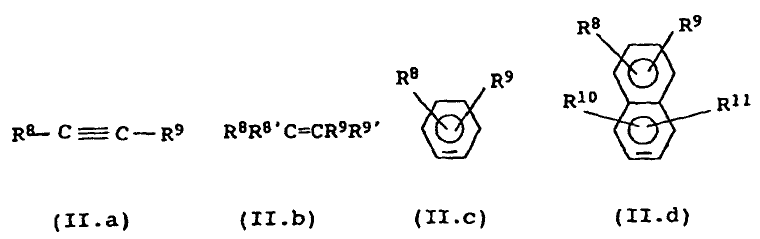 ou un précurseur approprié pour la formation d'un composé des formules II.c ou II.d, selon une cycloaddition [4+2] (réaction de Diels-Alder), et
fonctionnalisation des restes Y^{a} et/ou Y^{b} en les restes Y¹ et/ou Y².

9. Catalyseur, comprenant au moins un complexe d'un métal du groupe secondaire VIII avec au moins un ligand, qui est choisi parmi les composés de la formule générale I, comme défini selon l'une quelconque des revendications 1 à 7.

10. Catalyseur selon la revendication 9, **caractérisé en ce que** le métal du groupe secondaire VIII est choisi parmi cobalt, ruthénium, iridium, rhodium, nickel, palladium et platine.

11. Catalyseur selon l'une quelconque des revendications 9 et 10, qui présente en outre, au moins un autre ligand, choisi parmi les halogénures, les amines, les carboxylates, l'acétylacétonate, les aryl- ou alkylsulfonates, les hydrures, CO, les oléfines, les diènes, les cyclooléfines, les nitriles, les hétérocycles azotés, les aromatiques et hétéroaromatiques, les éthers, PF₃, les phospholènes, les phosphabenzènes ainsi que les ligands mono-, bi- et polyvalents de phosphine, phosphinite, phosphonite, phosphoramidite et phosphite.

12. Procédé d'hydroformylation de composés qui contiennent au moins une double liaison éthyléniquement insaturée, par réaction avec du monoxyde de carbone et de l'hydrogène en présence d'un catalyseur d'hydroformylation, **caractérisé en ce que** l'on met en oeuvre comme catalyseur d'hydroformylation, un catalyseur selon l'une quelconque des revendications 9 à 11.

13. Procédé d'hydrocyanation de composés qui contiennent au moins une double liaison éthyléniquement insaturée, par réaction avec le cyanure d'hydrogène en présence d'un catalyseur d'hydrocyanation, **caractérisé en ce que** l'on met en oeuvre comme catalyseur d'hydrocyanation, un catalyseur selon l'une quelconque des revendications 9 à 11.

14. Procédé selon l'une quelconque des revendications 12 ou 13, **caractérisé en ce que** le catalyseur d'hydroformylation ou le catalyseur d'hydrocyanation est préparé in situ, où on fait réagir au moins un composé de la formule générale I, comme défini dans les revendications 1 à 7, un composé ou un complexe d'un métal du groupe secondaire VIII et le cas échéant, un agent d'activation dans un solvant inerte, dans des conditions d'hydroformylation ou des conditions d'hydrocyanation.

15. Procédé de carbonylation de composés qui contiennent au moins une double liaison éthyléniquement insaturée, par réaction avec du monoxyde de carbone et au moins un composé ayant un radical nucléophile, en présence d'un catalyseur de carbonylation, **caractérisé en ce que** l'on met en oeuvre comme catalyseur de carbonylation, un catalyseur selon l'une quelconque des revendications 9 à 11.

16. Utilisation de catalyseurs, comprenant au moins un complexe d'un métal du groupe secondaire VIII avec au moins un ligand, qui est choisi parmi les composés de la formule générale I, comme défini selon l'une quelconque des revendications 1 à 7.
